# EUROPEAN PATENT SPECIFICATION

(11) **EP 2 809 777 B1**
(45) Date of publication and mention of the grant of the patent: **29.05.2019**
(21) Application number: 13702014.5
(22) Date of filing: 28.01.2013
(51) Int. Cl.: C12N 9/02, C12P 35/00, C12P 37/00

(54) **MBTH-LIKE PROTEINS IN THE PRODUCTION OF SEMI SYNTHETIC ANTIBIOTICS**
MBTH-ARTIGE PROTEINE IN DER HERSTELLUNG VON SEMISYNTHETISCHEN ANTIBIOTIKA
PROTÉINES DE TYPE MBTH DANS LA PRODUCTION D'ANTIBIOTIQUES SEMI-SYNTHETIQUE

(30) Priority: 31.01.2012 EP 12153225
(43) Date of publication of application: 10.12.2014
(73) Proprietor: Centrient Pharmaceuticals Netherlands B.V., 2613 AX Delft (NL)
(72) Inventor: MUELLER, Ulrike Maria, NL-6100 AC Echt (NL); BOER, Rémon, NL-6100 AC Echt (NL); BOVENBERG, Roelof, Ary, Lans, NL-6100 AC Echt (NL)
(74) Representative: de Pauw, Elmar Sebastian David
(86) International application number: PCT/EP2013/051544
(87) International publication number: WO 2013/113646

(56) References cited:
- WO-A2-2008/040731
- RICHARD H BALTZ: "Function of MbtH homologs in nonribosomal peptide biosynthesis and applications in secondary metabolite discovery", JOURNAL OF INDUSTRIAL MICROBIOLOGY & BIOTECHNOLOGY ; OFFICIAL JOURNAL OF THE SOCIETY FOR INDUSTRIAL MICROBIOLOGY, SPRINGER, BERLIN, DE, vol. 38, no. 11, 9 August 2011 (2011-08-09), pages 1747-1760, XP019967936, ISSN: 1476-5535, DOI: 10.1007/S10295-011-1022-8 cited in the application
- E.A. FELNAGLE ET AL: "MbtH-Like Proteins as Integral Components of Bacterial Nonribosomal Peptide Synthetases", BIOCHEMISTRY, vol. 49, 2010, pages 8815-8817, XP002676435,
- WENJUM ZHANG ET AL: "Activation of the Pacidamycin PacL Adenylation Domain by MbtH-Like Proteins", BIOCHEMISTRY, vol. 49, 2010, pages 9946-9947, XP002676436,
- B. BOLL ET AL: "Role of MbtH-like Proteins in the Adenylation of Tyrosine during Aminocoumarin and Vancomycin Biosynthesis", THE JOURNAL OF BIOLOGICAL CHEMISTRY, vol. 286, no. 42, 21 October 2011 (2011-10-21), pages 36281-36290, XP002676437,

### SEQUENCE LISTING

<110> DSM Sinochem Pharmaceuticals Netherlands B.V.
<120> MBTH-LIKE PROTEINS IN THE PRODUCTION OF SEMI SYNTHETIC ANTIBIOTICS
<130> 28457-WO-PCT
<150> EP12153225.3
   <151> 2012-01-31
<160> 35
<170> BiSSAP 1.2
<210> 1
   <211> 503
   <212> PRT
   <213> Actinoplanes teichomyceticus
<400> 1
   Met Asn Ser Ala Ala Gln Ala Thr Ser Thr Val Pro Glu Leu Leu Ala
   1               5                   10                  15
   Arg Gln Val Thr Arg Ala Pro Asp Ala Val Ala Val Val Asp Arg Asp
               20                  25                  30
   Arg Val Leu Thr Tyr Arg Glu Leu Asp Glu Leu Ala Gly Arg Leu Ser
           35                  40                  45
   Gly Arg Leu Ile Gly Arg Gly Val Arg Arg Gly Asp Arg Val Ala Val
       50                  55                  60
   Leu Leu Asp Arg Ser Ala Asp Leu Val Val Thr Leu Leu Ala Ile Trp
   65                  70                  75                  80
   Lys Ala Gly Ala Ala Tyr Val Pro Val Asp Ala Gly Tyr Pro Ala Pro
                   85                  90                  95
   Arg Val Ala Phe Met Val Ala Asp Ser Gly Ala Ser Arg Met Val Cys
               100                 105                 110
   Ser Ala Ala Thr Arg Asp Gly Val Pro Glu Gly Ile Glu Ala Ile Val
           115                 120                 125
   Val Thr Asp Glu Glu Ala Phe Glu Ala Ser Ala Ala Gly Ala Arg Pro
       130                 135                 140
   Gly Asp Leu Ala Tyr Val Met Tyr Thr Ser Gly Ser Thr Gly Ile Pro
   145                 150                 155                 160
   Lys Gly Val Ala Val Pro His Arg Ser Val Ala Glu Leu Ala Gly Asn
                   165                 170                 175
   Pro Gly Trp Ala Val Glu Pro Gly Asp Ala Val Leu Met His Ala Pro
               180                 185                 190
   Tyr Ala Phe Asp Ala Ser Leu Phe Glu Ile Trp Val Pro Leu Val Ser
           195                 200                 205
   Gly Gly Arg Val Val Ile Ala Glu Pro Gly Pro Val Asp Ala Arg Arg
       210                 215                 220
   Leu Arg Glu Ala Ile Ser Ser Gly Val Thr Arg Ala His Leu Thr Ala
   225                 230                 235                 240
   Gly Ser Phe Arg Ala Val Ala Glu Glu Ser Pro Glu Ser Phe Ala Gly
                   245                 250                 255
   Leu Arg Glu Val Leu Thr Gly Gly Asp Val Val Pro Ala His Ala Val
               260                 265                 270
   Ala Arg Val Arg Ser Ala Cys Pro Arg Val Arg Ile Arg His Leu Tyr
           275                 280                 285
   Gly Pro Thr Glu Thr Thr Leu Cys Ala Thr Trp His Leu Leu Glu Pro
       290                 295                 300
   Gly Asp Glu Ile Gly Pro Val Leu Pro Ile Gly Arg Pro Leu Pro Gly
   305                 310                 315                 320
   Arg Arg Ala Gln Val Leu Asp Ala Ser Leu Arg Ala Val Ala Pro Gly
                   325                 330                 335
   Val Ile Gly Asp Leu Tyr Leu Ser Gly Ala Gly Leu Ala Asp Gly Tyr
               340                 345                 350
   Leu Arg Arg Ala Gly Leu Thr Ala Glu Arg Phe Val Ala Asp Pro Ser
           355                 360                 365
   Ala Pro Gly Ala Arg Met Tyr Arg Thr Gly Asp Leu Ala Gln Trp Thr
       370                 375                 380
   Ala Asp Gly Ala Leu Leu Phe Ala Gly Arg Ala Asp Asp Gln Val Lys
   385                 390                 395                 400
   Val Arg Gly Phe Arg Ile Glu Pro Ala Glu Val Glu Ala Ala Leu Thr
                   405                 410                 415
   Ala Gln Pro Gly Val His Glu Ala Val Val Arg Ala Val Asp Gly Arg
               420                 425                 430
   Leu Val Gly Tyr Val Val Ala Glu Gly Asp Ala Glu Pro Ala Val Leu
           435                 440                 445
   Arg Glu Arg Val Gly Ala Val Leu Pro Glu Tyr Met Val Pro Ala Ala
       450                 455                 460
   Val Ile Thr Leu Asp Ala Leu Pro Leu Thr Gly Asn Gly Lys Val Asp
   465                 470                 475                 480
   Arg Ala Ala Leu Pro Ala Pro Val Phe Ala Ala Asp Ala Pro Gly Arg
                   485                 490                 495
   Glu Pro Gly Thr Glu Ala Glu
               500
<210> 2
   <211> 504
   <212> PRT
   <213> Nonomurea sp. ATCC39727
<400> 2
   Met Ser Ala Gly Thr Arg Ala Thr Pro Thr Thr Val Leu Asp Leu Phe
   1               5                   10                  15
   Ala Arg Gln Val Gly Arg Ala Pro Asp Ala Val Ala Leu Val Asp Gly
               20                  25                  30
   Asp Arg Val Leu Thr Tyr Arg Arg Leu Asp Glu Leu Ala Gly Ala Leu
           35                  40                  45
   Ser Gly Arg Leu Ile Gly Arg Gly Val Gly Arg Gly Asp Arg Val Ala
       50                  55                  60
   Val Met Met Asp Arg Ser Ala Asp Leu Val Val Thr Leu Leu Ala Val
   65                  70                  75                  80
   Trp Gln Ala Gly Ala Ala Tyr Val Pro Val Asp Ala Ala Leu Pro Ala
                   85                  90                  95
   Arg Arg Val Ala Phe Met Val Ala Asp Ser Gly Ala Cys Leu Met Val
               100                 105                 110
   Cys Ser Glu Ala Thr Arg Asp Ala Val Pro Gln Gly Val Glu Ser Ile
           115                 120                 125
   Ala Leu Thr Gly Glu Gly Gly Cys Gly Thr Ser Ala Val Thr Val Asp
       130                 135                 140
   Pro Gly Asp Leu Ala Tyr Val Met Tyr Thr Ser Gly Ser Thr Gly Thr
   145                 150                 155                 160
   Pro Lys Gly Val Ala Val Pro His Arg Ser Val Ala Glu Leu Thr Gly
                   165                 170                 175
   Asn Pro Gly Trp Gly Val Glu Pro Gly Glu Ala Val Leu Met His Ala
               180                 185                 190
   Pro Tyr Thr Phe Asp Ala Ser Leu Phe Glu Ile Trp Val Pro Leu Val
           195                 200                 205
   Ser Gly Ala Arg Val Val Ile Ala Ala Pro Gly Ala Val Asp Ala Arg
       210                 215                 220
   Arg Leu Arg Glu Ala Val Ala Ala Gly Val Thr Arg Val His Leu Thr
   225                 230                 235                 240
   Ala Gly Ser Phe Arg Ala Val Ala Glu Glu Ser Pro Glu Ser Phe Ala
                   245                 250                 255
   His Phe Arg Glu Val Leu Thr Gly Gly Asp Val Val Pro Ala Tyr Ala
               260                 265                 270
   Val Gln Lys Val Arg Ala Ala Cys Pro His Val Arg Ile Arg His Leu
           275                 280                 285
   Tyr Gly Pro Thr Glu Thr Thr Leu Cys Ala Thr Trp Gln Leu Leu Glu
       290                 295                 300
   Pro Gly Asp Val Val Gly Pro Val Leu Pro Ile Gly Arg Pro Leu Pro
   305                 310                 315                 320
   Gly Arg Arg Ala Trp Val Leu Asp Ala Ser Leu Arg Pro Val Glu Pro
                   325                 330                 335
   Gly Val Val Gly Asp Leu Tyr Leu Ser Gly Ala Gly Leu Ala Asp Gly
               340                 345                 350
   Tyr Leu Asp Arg Ala Gly Leu Thr Ala Glu Arg Phe Val Ala Asp Pro
           355                 360                 365
   Ser Ala Ala Gly Arg Arg Met Tyr Arg Thr Gly Asp Leu Ala Gln Trp
       370                 375                 380
   Thr Ala Asp Gly Glu Leu Leu Phe Ala Gly Arg Ala Asp Asp Gln Val
   385                 390                 395                 400
   Lys Val Arg Gly Phe Arg Ile Glu Pro Gly Glu Val Glu Ala Ala Leu
                   405                 410                 415
   Thr Ala Gln Pro His Val Arg Glu Ala Val Val Val Ala Ile Asp Gly
               420                 425                 430
   Arg Leu Ile Gly Tyr Val Val Ala Asp Gly Asp Val Asp Pro Val Leu
           435                 440                 445
   Met Arg Arg Arg Leu Ala Ala Ser Leu Pro Glu Tyr Met Ile Pro Ala
       450                 455                 460
   Ala Leu Val Thr Leu Asp Ala Leu Pro Leu Thr Gly Ser Gly Lys Val
   465                 470                 475                 480
   Asp Arg Arg Ala Leu Pro Glu Pro Asp Phe Ala Ser Ala Ala Pro Arg
                   485                 490                 495
   Arg Glu Pro Gly Thr Glu Pro Glu
               500
<210> 3
   <211> 500
   <212> PRT
   <213> Streptomyces toyocaensis
<400> 3
   Met Asn Ser Val Leu Ser Thr Pro Thr Val Pro Glu Leu Phe Ala Arg
   1               5                   10                  15
   Gln Ala Glu Arg Thr Pro Glu Ala Val Ala Val Val Asp Gly Asp Arg
               20                  25                  30
   Phe Val Thr Tyr Arg Gln Leu Asp Glu Leu Ala Gly Arg Leu Ala Gly
           35                  40                  45
   Arg Leu Ile Gly Arg Gly Val Arg Arg Gly Asp Arg Val Ala Val Leu
       50                  55                  60
   Met Glu Arg Ser Ala Asp Leu Val Val Thr Leu Leu Ala Val Trp Lys
   65                  70                  75                  80
   Ala Gly Ala Ala Tyr Val Pro Val Asp Ala Ala His Pro Ala Pro Arg
                   85                  90                  95
   Val Ala Phe Val Val Ala Asp Ser Gly Ala Ser Leu Met Ala Cys Ser
               100                 105                 110
   Ala Ala Thr Ala Gly Arg Val Pro Glu Gly Val Glu Pro Val Val Val
           115                 120                 125
   Thr Asp Glu Gly Arg Gly Asp Ala Ser Ala Val Pro Val Ser Pro Gly
       130                 135                 140
   Asp Leu Ala Tyr Val Met Tyr Thr Ser Gly Ser Thr Gly Thr Pro Lys
   145                 150                 155                 160
   Gly Val Ala Val Pro His Arg Ser Val Ala Glu Leu Ala Gly Asn Pro
                   165                 170                 175
   Gly Trp Ala Val Lys Pro Gly Asp Ala Ile Leu Met His Ala Pro His
                   180                 185                 190
   Ala Phe Asp Ala Ser Leu Phe Glu Ile Trp Val Pro Leu Val Ser Gly
           195                 200                 205
   Ala Arg Val Val Ile Ala Glu Pro Gly Ala Val Asp Ala Arg Arg Leu
       210                 215                 220
   Arg Glu Ala Ile Ala Ala Gly Val Thr Lys Val His Leu Thr Ala Gly
   225                 230                 235                 240
   Ser Phe Arg Ala Leu Ala Glu Glu Ser Ser Glu Ser Phe Ala Gly Leu
                   245                 250                 255
   Gln Glu Val Leu Thr Gly Gly Asp Val Val Pro Ala His Ala Val Glu
               260                 265                 270
   Lys Val Arg Lys Ala Val Pro Gln Ala Arg Ile Arg His Leu Tyr Gly
           275                 280                 285
   Pro Thr Glu Thr Thr Leu Cys Ala Thr Trp His Leu Leu Gln Pro Ser
       290                 295                 300
   Glu Ala Leu Gly Pro Val Leu Pro Ile Gly Arg Pro Leu Pro Gly Arg
   305                 310                 315                 320
   Arg Ala Gln Val Leu Asp Ala Ser Leu Arg Pro Leu Pro Pro Gly Val
                   325                 330                 335
   Val Gly Asp Leu Tyr Leu Ser Gly Ala Gly Leu Ala Asp Gly Tyr Leu
               340                 345                 350
   Asp Arg Ala Ala Leu Thr Ala Glu Arg Phe Val Ala Asp Pro Ser Val
           355                 360                 365
   Pro Gly Gly Arg Met Tyr Arg Thr Gly Asp Leu Val Gln Trp Thr Ala
       370                 375                 380
   Asp Gly Glu Leu Leu Phe Val Gly Arg Ala Asp Asp Gln Val Lys Ile
   385                 390                 395                 400
   Arg Gly Phe Arg Ile Glu Pro Gly Glu Ile Glu Ala Ala Leu Thr Ala
                   405                 410                 415
   Gln Pro Asp Val His Glu Ala Val Val Val Ala Ile Asp Gly Arg Leu
               420                 425                 430
   Ile Gly Tyr Ala Val Thr Asp Val Asp Pro Val Val Leu Arg Glu Arg
           435                 440                 445
   Leu Gly Ala Thr Leu Pro Glu Tyr Met Val Pro Ala Val Val Ile Thr
       450                 455                 460
   Leu Asp Gly Leu Pro Leu Thr Arg Asn Gly Lys Val Asp Arg Ala Ala
   465                 470                 475                 480
   Leu Pro Ala Pro Val Phe Gly Thr Asn Ala Ala Gly Arg Glu Pro Ala
                   485                 490                 495
   Thr Glu Ala Glu
               500
<210> 4
   <211> 540
   <212> PRT
   <213> Amycolatopsis orientalis
<400> 4
   Leu Pro Val Gly Arg Leu Gly Val Thr Ser Glu Pro Ala Arg Ala Ser
   1               5                   10                  15
   Val Val Glu Arg Trp Asn Ser Thr Gly Glu Ala Ala Asn Arg Thr Ser
               20                  25                  30
   Val Leu Glu Leu Phe Arg Gln Gln Ala Asp Ala Ser Pro Asp Ala Val
           35                  40                  45
   Ala Val Met Asp Ala Ala Arg Thr Leu Ser Tyr Ala Asp Leu Asp Arg
       50                  55                  60
   Glu Ser Asp Arg Leu Ala Gly Tyr Leu Ala Ala Met Gly Val Arg Arg
   65                  70                  75                  80
   Gly Asp Arg Val Gly Val Val Met Glu Arg Gly Thr Asp Leu Phe Val
                   85                  90                  95
   Ala Leu Leu Ala Val Trp Lys Ala Gly Ala Ala Gln Val Pro Val Asn
               100                 105                 110
   Val Asp Tyr Pro Ala Glu Arg Ile Glu Arg Met Leu Ala Asp Ala Gly
   115 120 125
   Ala Ser Val Ala Val Cys Leu Glu Ala Thr Arg Lys Ala Val Pro Asp
   130 135 140
   Gly Val Glu Pro Val Val Met Asp Val Pro Ala Ile Asp Gly Val Arg
   145 150 155 160
   His Glu Ala Pro Gln Val Thr Val Gly Ala His Asp Leu Ala Tyr Val
   165 170 175
   Met Tyr Thr Ser Gly Ser Thr Gly Val Pro Lys Gly Val Ala Val Pro
   180 185 190
   His Gly Ser Val Ala Ala Leu Ala Ser Asp Pro Gly Trp Ser Gln Gly
   195 200 205
   Pro Asp Asp Cys Val Leu Leu His Ala Ser His Ala Phe Asp Ala Ser
   210 215 220
   Leu Val Glu Ile Trp Val Pro Leu Val Asn Gly Ser Arg Val Met Val
   225 230 235 240
   Ala Glu Pro Gly Ala Val Asp Ala Glu Arg Leu Arg Glu Ala Ile Ser
   245 250 255
   Arg Gly Val Thr Thr Val His Leu Thr Ala Gly Ala Phe Arg Ala Val
   260 265 270
   Ala Glu Glu Ser Pro Asp Ser Phe Thr Gly Leu Arg Glu Ile Leu Thr
   275 280 285
   Gly Gly Asp Ala Val Pro Leu Ala Ser Val Val Arg Met Arg Arg Ala
   290 295 300
   Cys Pro Asp Val Arg Val Arg Gln Leu Tyr Gly Pro Thr Glu Ile Thr
   305 310 315 320
   Leu Cys Ala Thr Trp His Val Ile Glu Pro Gly Ala Glu Thr Gly Asp
   325 330 335
   Thr Leu Pro Ile Gly Arg Pro Leu Ala Gly Arg Gln Ala Tyr Val Leu
   340 345 350
   Asp Ala Phe Leu Gln Pro Val Ala Pro Asn Val Thr Gly Glu Leu Tyr
   355 360 365
   Ile Ala Gly Ala Gly Leu Ala His Gly Tyr Leu Gly Asn Asn Gly Ser
   370 375 380
   Thr Ser Glu Arg Phe Ile Ala Asn Pro Phe Ala Ser Gly Glu Arg Met
   385 390 395 400
   Tyr Arg Thr Gly Asp Leu Ala Arg Trp Thr Asp Gln Gly Glu Leu Leu
   405 410 415
   Phe Ala Gly Arg Ala Asp Ser Gln Val Lys Ile Arg Gly Tyr Arg Val
   420 425 430
   Glu Pro Gly Glu Ile Glu Val Ala Leu Thr Glu Val Pro His Val Ala
   435 440 445
   Gln Ala Val Val Val Ala Arg Glu Asp His Pro Gly Asp Lys Arg Leu
   450 455 460
   Ile Ala Tyr Val Thr Ala Glu Glu Gly Pro Ala Leu Ala Ala Asp Ala
   465 470 475 480
   Val Arg Glu His Leu Ala Ala Arg Met Pro Glu Phe Met Val Pro Ala
   485 490 495
   Val Val Leu Val Leu Asp Ser Phe Pro Leu Thr Leu Asn Gly Lys Ile
   500 505 510
   Asp Arg Ala Ala Leu Pro Ala Pro Glu Phe Thr Gly Lys Ala Ala Gly
   515 520 525
   Arg Glu Pro Arg Thr Glu Thr Glu Arg Val Leu Cys
   530 535 540
<210> 5
   <211> 539
   <212> PRT
   <213> Amycolatopsis balhimycina
<400> 5
   Val Gly Arg Leu Gly Val Thr Ser Glu Pro Thr Arg Ala Ala Val Val
   1               5                   10                  15
   Glu Arg Trp Asn Ser Thr Gly Glu Ala Ala Ala Glu Thr Ser Val Leu
               20                  25                  30
   Glu Leu Phe Arg Arg Gln Ala Gly Ala Ser Pro Asp Ala Val Ala Val
           35                  40                  45
   Val Ala Gly Glu Arg Thr Leu Ser Tyr Ala Asp Leu Asp Arg Glu Ser
       50                  55                  60
   Asp Arg Leu Ala Gly His Leu Ala Gly Ile Gly Val Gly Arg Gly Asp
   65                  70                  75                  80
   Arg Val Gly Val Val Met Thr Arg Gly Ala Asp Leu Phe Val Ala Leu
                   85                  90                  95
   Leu Gly Val Trp Lys Ala Gly Ala Ala Gln Val Pro Val Asn Val Asp
               100                 105                 110
   Tyr Pro Ala Glu Arg Ile Glu Arg Met Leu Ala Asp Val Gly Ala Ser
           115                 120                 125
   Val Ala Val Cys Val Glu Ala Thr Arg Lys Ala Val Pro Asp Gly Val
       130                 135                 140
   Glu Pro Val Val Val Asp Leu Pro Val Ile Gly Gly Val Arg Pro Glu
   145                 150                 155                 160
   Ala Pro Pro Val Thr Val Gly Ala His Asp Val Ala Tyr Val Met Tyr
                   165                 170                 175
   Thr Ser Gly Ser Thr Gly Val Pro Lys Ala Val Ala Val Pro His Gly
               180                 185                 190
   Ser Val Ala Ala Leu Ala Ser Asp Pro Gly Trp Ser Gln Gly Pro Gly
           195                 200                 205
   Asp Cys Val Leu Leu His Ala Ser His Ala Phe Asp Ala Ser Leu Val
       210                 215                 220
   Glu Ile Trp Val Pro Leu Val Ser Gly Ala Arg Val Leu Val Ala Glu
   225                 230                 235                 240
   Pro Gly Thr Val Asp Ala Glu Arg Leu Arg Glu Ala Val Ser Arg Gly
                   245                 250                 255
   Val Thr Thr Val His Leu Thr Ala Gly Ala Phe Arg Ala Val Ala Glu
               260                 265                 270
   Glu Ser Pro Asp Ser Phe Ile Gly Leu Arg Glu Ile Leu Thr Gly Gly
           275                 280                 285
   Asp Ala Val Pro Leu Ala Ser Val Val Arg Met Arg Gln Ala Cys Pro
       290                 295                 300
   Asp Val Arg Val Arg Gln Leu Tyr Gly Pro Thr Glu Ile Thr Leu Cys
   305                 310                 315                 320
   Ala Thr Trp Leu Val Leu Glu Pro Gly Ala Ala Thr Gly Asp Val Leu
                   325                 330                 335
   Pro Ile Gly Arg Pro Leu Ala Gly Arg Gln Ala Tyr Val Leu Asp Ala
               340                 345                 350
   Phe Leu Gln Pro Val Ala Pro Asn Val Thr Gly Glu Leu Tyr Leu Ala
           355                 360                 365
   Gly Ala Gly Leu Ala His Gly Tyr Leu Gly Asn Thr Ala Ala Thr Ser
       370                 375                 380
   Glu Arg Phe Val Ala Asn Pro Phe Ser Gly Gly Gly Arg Met Tyr Arg
   385                 390                 395                 400
   Thr Gly Asp Leu Ala Arg Trp Thr Asp Gln Gly Glu Leu Val Phe Ala
                   405                 410                 415
   Gly Arg Ala Asp Ser Gln Val Lys Ile Arg Gly Tyr Arg Val Glu Pro
               420                 425                 430
   Gly Glu Val Glu Val Ala Leu Thr Glu Val Pro His Val Ala Gln Ala
           435                 440                 445
   Val Val Val Ala Arg Glu Gly Gln Pro Gly Glu Lys Arg Leu Ile Ala
       450                 455                 460
   Tyr Val Thr Ala Glu Ala Gly Ser Ala Leu Glu Ser Ala Ala Val Arg
   465                 470                 475                 480
   Ala His Leu Ala Thr Arg Leu Pro Glu Phe Met Val Pro Ser Val Val
                   485                 490                 495
   Val Val Leu Glu Ser Phe Pro Leu Thr Leu Asn Gly Lys Ile Asp Arg
               500                 505                 510
   Ala Ala Leu Pro Ala Pro Glu Phe Ala Gly Lys Ala Ala Gly Arg Glu
           515                 520                 525
   Pro Arg Thr Glu Ala Glu Arg Val Leu Cys Gly
       530                 535
<210> 6
   <211> 539
   <212> PRT
   <213> uncultured soil bacterium
<400> 6
   Ser Thr Val Ala Asp Val Asp Val Thr Ser Ala Ala Glu Arg Ala Leu
   1               5                   10                  15
   Val Val Asp Glu Trp Gly Ala Ala Ala Glu Ala Ala Pro Ser Arg Leu
               20                  25                  30
   Ala Leu Glu Leu Phe Asp Gly Gln Val Glu Ser Arg Arg Asp Ala Ile
           35                  40                  45
   Ala Val Val Asp Arg Asp Gln Ala Met Ser Tyr Gly Val Leu Ala Glu
       50                  55                  60
   Asp Ala Glu Arg Leu Ala Gly Tyr Leu Asn Gly Arg Gly Val Arg Arg
   65                  70                  75                  80
   Gly Asp Arg Val Ala Val Val Val Glu Arg Ser His Asp Leu Ile Ala
                   85                  90                  95
   Thr Leu Leu Ala Val Trp Lys Ala Gly Ala Ala Tyr Val Pro Val Asp
               100                 105                 110
   Pro Ala Tyr Pro Leu Glu Arg Val Lys Phe Met Leu Ala Asp Ala Asp
           115                 120                 125
   Pro Ala Ala Val Val Cys Thr Ala Gly Tyr Arg Asp Ser Val Leu Asp
       130                 135                 140
   Gly Gly Leu Asp Pro Ile Val Leu Asp Asp Pro Gln Thr Arg Gln Ala
   145                 150                 155                 160
   Val Ser Glu Cys Ser Arg Leu Ser Val Gly Thr Thr Ala Asp Asp Val
                   165                 170                 175
   Ala Tyr Val Met Tyr Thr Ser Gly Ser Thr Gly Thr Pro Lys Gly Val
               180                 185                 190
   Ala Val Ser His Gly Asn Val Ala Ala Leu Val Gly Glu Pro Gly Trp
           195                 200                 205
   Arg Val Gly Pro Asp Asp Ala Val Leu Met His Ala Ser His Ala Phe
       210                 215                 220
   Asp Ile Ser Leu Phe Glu Met Trp Val Pro Leu Val Ser Gly Ala Arg
   225                 230                 235                 240
   Val Val Leu Ala Gly Ser Gly Ala Val Asp Gly Ala Ala Leu Ala Ala
                   245                 250                 255
   Tyr Val Ala Asp Gly Val Thr Ala Ala His Leu Thr Ala Gly Ala Phe
               260                 265                 270
   Arg Val Leu Ala Glu Glu Ser Pro Glu Ser Val Ala Gly Leu Arg Glu
           275                 280                 285
   Val Leu Thr Gly Gly Asp Ala Val Pro Leu Ala Ala Val Glu Arg Val
       290                 295                 300
   Arg Arg Thr Cys Pro Asp Val Arg Val Arg His Leu Tyr Gly Pro Thr
   305                 310                 315                 320
   Glu Ala Thr Leu Cys Ala Thr Trp Leu Leu Leu Glu Pro Gly Asp Glu
                   325                 330                 335
   Thr Gly Pro Val Leu Pro Ile Gly Arg Pro Leu Ala Gly Arg Arg Val
               340                 345                 350
   Tyr Val Leu Asp Gly Phe Leu Arg Pro Val Pro Pro Gly Val Ala Gly
           355                 360                 365
   Glu Leu Tyr Val Ala Gly Ala Gly Val Ala Gln Gly Tyr Leu Glu Arg
       370                 375                 380
   Pro Ala Leu Thr Ala Glu Arg Phe Val Ala Asp Pro Phe Val Ala His
   385                 390                 395                 400
   Gly Arg Met Tyr Arg Thr Gly Asp Leu Ala Tyr Trp Thr Gly Lys Gly
                   405                 410                 415
   Ala Leu Ala Phe Ala Gly Arg Ala Asp Asp Gln Val Lys Ile Arg Gly
               420                 425                 430
   Tyr Arg Val Glu Pro Gly Glu Ile Glu Val Val Leu Ala Gly Leu Pro
           435                 440                 445
   Gly Val Gly Gln Ala Val Val Leu Ala Arg Asp Glu His Leu Ile Gly
       450                 455                 460
   Tyr Ala Val Ala Glu Ala Gly His Glu Leu Asp Pro Val Arg Leu Arg
   465                 470                 475                 480
   Glu Gln Leu Ala Asp Thr Leu Pro Glu Phe Met Val Pro Ala Ala Val
                   485                 490                 495
   Leu Val Leu Gly Glu Leu Pro Leu Thr Val Asn Gly Lys Val Asp Arg
               500                 505                 510
   Gln Ala Leu Pro Gly Pro Asp Phe Ala Ser Lys Ala Ala Gly Arg Ala
           515                 520                 525
   Pro Ala Thr Asp Ala Glu Arg Val Leu Cys Gly
       530                 535
<210> 7
   <211> 535
   <212> PRT
   <213> Actinoplanes teichomyceticus
<400> 7
   Leu Thr Val Ala Ala Ile Asp Val Thr Ser Ala Ala Glu Arg Asp Arg
   1               5                   10                  15
   Val Ala Arg Trp Gly Ala Ala Val Gly Ala Arg Pro Asp Arg Leu Ala
               20                  25                  30
   Leu Asp Leu Phe Ala Arg Gln Val Ala Gln Arg Pro Asp Glu Val Ala
           35                  40                  45
   Val Ala Asp Gly Asp Arg Val Met Ser Phe Gly Glu Leu Ala Glu Arg
       50                  55                  60
   Ala Asp Arg Leu Ala Gly His Leu Ser Ala Arg Gly Val Arg Arg Gly
   65                  70                  75                  80
   Asp Arg Val Ala Val Val Met Glu Arg Ser Gly Glu Leu Ile Ala Thr
                   85                  90                  95
   Leu Leu Ala Val Trp Arg Ala Gly Ala Ala Phe Val Pro Val Asp Pro
               100                 105                 110
   Ala Tyr Pro Ala Glu Arg Val Lys Phe Leu Leu Thr Asp Ala Glu Pro
           115                 120                 125
   Val Ala Ala Val Cys Thr Ala Ala Phe Arg Ala Ala Val Leu Asp Gly
       130                 135                 140
   Gly Leu Glu Ala Ile Val Val Asp Asp Pro Gly Thr Trp Pro Ala Val
   145                 150                 155                 160
   Ala Pro Cys Pro Pro Val Pro Thr Gly Pro Asp Asp Leu Ala Tyr Val
                   165                 170                 175
   Met Tyr Thr Ser Gly Ser Thr Gly Thr Pro Lys Gly Val Ala Val Ser
               180                 185                 190
   His Gly Asp Val Ala Ala Leu Val Gly Asp Pro Gly Trp Arg Thr Gly
           195                 200                 205
   Pro Gly Asp Thr Val Leu Met His Ala Ser His Ala Phe Asp Ile Ser
       210                 215                 220
   Leu Phe Glu Ile Trp Val Pro Leu Leu Ser Gly Ala Arg Val Met Ile
   225                 230                 235                 240
   Ala Gly Pro Gly Ala Val Asp Gly Ala Ala Leu Ala Ala Gln Val Ala
                   245                 250                 255
   Ala Gly Val Thr Ala Ala His Leu Thr Ala Gly Ala Phe Arg Val Leu
               260                 265                 270
   Ala Glu Glu Ser Pro Glu Ser Val Ala Gly Leu Arg Glu Val Leu Thr
           275                 280                 285
   Gly Gly Asp Ala Val Pro Leu Ala Ala Val Glu Arg Val Arg Arg Ala
       290                 295                 300
   Cys Pro Asp Val Arg Val Arg His Leu Tyr Gly Pro Thr Glu Thr Thr
   305                 310                 315                 320
   Leu Cys Ala Thr Trp Trp Leu Leu Glu Pro Gly Asp Glu Thr Gly Pro
                   325                 330                 335
   Val Leu Pro Ile Gly Arg Pro Leu Ala Gly Arg Arg Val Tyr Val Leu
               340                 345                 350
   Asp Ala Phe Leu Arg Pro Leu Pro Pro Gly Thr Thr Gly Glu Leu Tyr
           355                 360                 365
   Val Ala Gly Ala Gly Val Ala Gln Gly Tyr Leu Gly Arg Pro Ala Leu
       370                 375                 380
   Thr Ala Glu Arg Phe Val Ala Asp Pro Phe Ala Pro Gly Gly Arg Met
   385                 390                 395                 400
   Tyr Arg Thr Gly Asp Leu Ala Tyr Trp Thr Glu Gln Gly Thr Leu Ala
                   405                 410                 415
   Phe Ala Gly Arg Ala Asp Asp Gln Val Lys Ile Arg Gly Tyr Arg Val
               420                 425                 430
   Glu Pro Gly Glu Val Glu Ala Val Leu Gly Gly Leu Pro Gly Val Ala
           435                 440                 445
   Gln Ala Val Val Cys Val Arg Gly Glu His Leu Ile Gly Tyr Val Val
       450                 455                 460
   Ala Glu Ala Gly Arg Asp Leu Asp Pro Glu Arg Leu Arg Ala Arg Leu
   465                 470                 475                 480
   Ala Ala Thr Leu Pro Glu Phe Met Val Pro Ala Ala Val Leu Val Leu
                   485                 490                 495
   Ala Asp Leu Pro Leu Thr Val Asn Gly Lys Val Asp Arg Pro Ala Leu
               500                 505                 510
   Pro Glu Pro Asp Phe Ala Ala Lys Ser Thr Gly Arg Ala Pro Ala Thr
           515                 520                 525
   Ala Ala Glu Arg Ile Leu Cys
       530                 535
<210> 8
   <211> 538
   <212> PRT
   <213> Streptomyces toyocaensis
<400> 8
   Leu Pro Val Gly Arg Leu Gly Val Thr Ser Asp Ala Thr Arg Thr Ser
   1               5                   10                  15
   Glu Val Glu Arg Trp Asn Ala Thr Gly Glu Ala Ala Gly Gly Ala Ser
               20                  25                  30
   Val Val Glu Leu Phe Arg Arg Arg Ser Ala Gly Thr Pro Asp Ala Val
           35                  40                  45
   Ala Val Val Asp Gly Asp Arg Thr Leu Ser Tyr Gly Asp Leu Asp Arg
       50                  55                  60
   Glu Ser Asp Arg Leu Ala Gly Arg Leu Ala Glu Thr Gly Val Arg Arg
   65                  70                  75                  80
   Gly Asp His Val Gly Val Val Leu Glu Arg Gly Ala Asp Leu Phe Val
                   85                  90                  95
   Ala Phe Leu Ala Val Trp Lys Ala Gly Ala Ala Tyr Val Pro Val His
               100                 105                 110
   Val Asp Tyr Pro Pro Val Arg Ile Glu Arg Met Leu Ala Asp Ala Gly
           115                 120                 125
   Val Thr Val Ala Val Cys Ala Glu Gly Thr Arg Asn Ala Val Pro Asp
       130                 135                 140
   Gly Leu Glu Pro Val Pro Val Asp Ala Pro Trp Ala Gly Glu Thr Arg
   145                 150                 155                 160
   His Glu Thr Pro Thr Val Thr Ala Arg Asp Ala Ala Tyr Val Met Tyr
                   165                 170                 175
   Thr Ser Gly Ser Thr Gly Glu Pro Lys Gly Ile Val Val Pro His Gly
               180                 185                 190
   Ser Val Ala Ala Leu Ala Gly Asp Pro Gly Trp Ala Leu Asp Ala Asp
           195                 200                 205
   Asp Cys Val Leu Met His Ala Ser His Ala Phe Asp Ala Ser Leu Phe
       210                 215                 220
   Glu Ile Trp Ala Pro Leu Val Arg Gly Ala Arg Val Met Val Ala Glu
   225                 230                 235                 240
   Pro Gly Ala Val Asp Thr Gln Arg Leu Arg Glu Ala Val Ala Arg Gly
                   245                 250                 255
   Val Thr Thr Val His Leu Thr Ala Gly Ser Phe Arg Val Leu Ala Glu
               260                 265                 270
   Glu Ser Pro Gly Ser Phe Asp Gly Leu Arg Glu Ile Leu Thr Gly Gly
           275                 280                 285
   Asp Val Val Pro Leu Ala Ser Val Ala Gln Leu Arg Arg Ala Cys Pro
       290                 295                 300
   Asp Val Arg Val Arg His Leu Tyr Gly Pro Thr Glu Thr Thr Leu Cys
   305                 310                 315                 320
   Gly Thr Trp His Leu Leu Glu Pro Gly Asp Glu Pro Gly Asp Val Leu
                   325                 330                 335
   Pro Ile Gly Arg Pro Leu Ala Gly Arg Arg Ala Tyr Val Leu Asp Ala
               340                 345                 350
   Phe Leu Gln Pro Val Ala Pro Asn Val Thr Gly Glu Leu Tyr Leu Ala
           355                 360                 365
   Gly Val Gly Leu Ala Leu Gly Tyr Leu Gly Ala Arg Gly Ala Thr Ser
       370                 375                 380
   Glu Arg Phe Val Ala Asp Pro Phe Val Pro Gly Glu Arg Met Tyr Arg
   385                 390                 395                 400
   Thr Gly Asp Leu Ala Arg Arg Asn Asp Arg Gly Glu Leu Leu Phe Ala
                   405                 410                 415
   Gly Arg Ala Asp Ala Gln Val Lys Ile Arg Gly Tyr Arg Val Glu Pro
               420                 425                 430
   Thr Glu Ile Glu Thr Val Leu Ala Glu Ala Pro Gln Val Ala Gln Thr
           435                 440                 445
   Val Val Val Ala Arg Glu Asp Gly Pro Gly Glu Lys Arg Leu Ile Ala
       450                 455                 460
   Tyr Ala Ile Ala Glu Pro Asp Gln Val Leu Asp Pro Glu Ala Leu Arg
   465                 470                 475                 480
   Glu His Leu Ala Ala Arg Leu Pro Glu Phe Met Val Pro Ala Ala Val
                   485                 490                 495
   Val Val Leu Asp Asp Phe Pro Leu Thr Ile Asn Gly Lys Ile Asp Arg
               500                 505                 510
   Glu Ala Leu Pro Ala Pro Glu Phe Ser Ala Lys Pro Ala Gly Arg Glu
           515                 520                 525
   Pro Arg Thr Glu Ala Glu Arg Val Leu Cys
       530                 535
<210> 9
   <211> 522
   <212> PRT
   <213> Streptomyces lavendulae
<400> 9
   Val Leu Val Gly Arg Val Gly Leu Val Gly Arg Leu Glu Arg Gly Leu
   1               5                   10                  15
   Val Val Glu Gly Trp Asn Ala Thr Ala Gly Asp Val Pro Ser Gly Ser
               20                  25                  30
   Ser Val Leu Glu Met Phe Arg Ala Arg Val Ala Gln Ala Pro Glu Ala
           35                  40                  45
   Val Ala Val Val Asp Gly Glu Arg Gln Val Ser Tyr Gly Glu Leu Asp
       50                  55                  60
   Ala Asp Ser Asn Arg Met Ala Ala Tyr Leu Gln Gly Arg Gly Val Gly
   65                  70                  75                  80
   Arg Gly Asp Arg Val Ala Val Arg Leu Glu Arg Ser Ile Asp Leu Ile
                   85                  90                  95
   Ala Ala Leu Leu Gly Val Trp Lys Ala Gly Ala Ala Tyr Val Pro Val
               100                 105                 110
   Asp Ser Ala Tyr Pro Ala Glu Arg Val Ala Phe Met Val Glu Asp Ser
           115                 120                 125
   Ala Pro Val Leu Thr Ile Asp Asp Pro Ser Val Val Thr Ala Glu Gly
       130                 135                 140
   Glu Pro Glu Val Val Glu Thr Ala Gly Gly Asp Ile Ala Tyr Val Met
   145                 150                 155                 160
   Tyr Thr Ser Gly Ser Thr Gly Thr Pro Lys Gly Val Ala Val Pro His
                   165                 170                 175
   Ala Ser Val Ala Ala Leu Val Gly Glu Pro Gly Trp Gly Val Gly Pro
               180                 185                 190
   Gly Asp Ala Val Leu Phe His Ala Pro His Ala Phe Asp Ile Ser Leu
           195                 200                 205
   Phe Glu Val Trp Val Pro Leu Ala Ser Gly Gly Arg Ile Val Val Ala
       210                 215                 220
   Glu Pro Ser Met Ala Val Asp Gly Ala Ala Val Arg Arg His Ile Ala
   225                 230                 235                 240
   Asp Gly Val Thr His Val His Val Thr Ala Gly Leu Phe Arg Val Leu
                   245                 250                 255
   Ala Glu Glu Ala Ser Asp Cys Phe Asp Gly Val His Glu Val Leu Thr
               260                 265                 270
   Gly Gly Asp Val Val Pro Leu Glu Ala Val Glu Arg Val Arg Ala Ala
           275                 280                 285
   Cys Pro Asp Val Arg Val Arg His Leu Tyr Gly Pro Thr Glu Val Ser
       290                 295                 300
   Leu Cys Ala Thr Trp His Leu Phe Glu Pro Gly Glu Glu Gln Gly Glu
   305                 310                 315                 320
   Val Leu Pro Leu Gly Arg Pro Leu Asn Asn Arg Gln Val Tyr Val Leu
                   325                 330                 335
   Asp Pro Phe Leu Gln Pro Val Pro Pro Gly Val Thr Gly Glu Leu Tyr
               340                 345                 350
   Val Ala Gly Ala Gly Leu Ala Arg Gly Tyr Leu Gly Arg Ala Gly Leu
           355                 360                 365
   Ser Ala Glu Arg Phe Val Ala Ser Pro Phe Ala Asp Gly Glu Arg Met
       370                 375                 380
   Tyr Arg Thr Gly Asp Leu Val Arg Trp Thr Thr Gly Val Glu Leu Val
   385                 390                 395                 400
   Phe Val Gly Arg Ala Asp Ala Gln Val Lys Ile Arg Gly Phe Arg Val
                   405                 410                 415
   Glu Leu Gly Glu Val Glu Ala Ala Leu Ala Ala Gln Pro Ala Val Ala
               420                 425                 430
   Gln Ala Val Val Val Ala Arg Glu Asp Arg Pro Gly Glu Lys Arg Leu
           435                 440                 445
   Val Gly Tyr Leu Val Pro Ser Gly Glu Glu Pro Asp Thr Glu Ala Val
       450                 455                 460
   His Ala Ser Leu Ala Asp Arg Leu Pro Glu Tyr Met Val Pro Ala Ala
   465                 470                 475                 480
   Leu Val Val Leu Asp Ala Leu Pro Leu Thr Val Asn Gly Lys Val Asp
                   485                 490                 495
   His Lys Ala Leu Pro Ala Pro Glu Phe Thr Ala Thr Ala Ser Arg Glu
               500                 505                 510
   Pro Arg Thr Ala Ala Glu Lys Leu Leu Cys
           515                 520
<210> 10
   <211> 1742
   <212> DNA
   <213> Artificial Sequence
<220>
   <221> source
   <222> 1..1742
   <223> /organism="Artificial Sequence" /note="Synthetic DNA" /mol_type="unassigned DNA"
<400> 10
   catatgagcg ctggcactag agcaaccccg accaccgtac tcgacctgtt cgcccgtcag      60
   gtcggccgtg cgccggacgc tgtcgcgctg gtggacggtg accgtgtcct gacctaccgc     120
   cgtctggatg agctggcggg tgcattgagc ggccgtctga ttggtcgtgg tgtcggccgt     180
   ggcgatcgcg tggccgtcat gatggaccgc agcgcggatc tggtcgttac cctgctggca     240
   gtttggcagg caggtgcggc gtacgttccg gtggacgcag cactgcctgc gcgtcgtgtg     300
   gccttcatgg tggcggatag cggtgcgtgt ctgatggtgt gctctgaggc gacccgcgat     360
   gccgtgccgc aaggtgttga gagcatcgca ctgaccggcg aaggtggttg tggtactagc     420
   gcggtcacgg tggacccagg cgacctggcc tatgtgatgt acacttccgg ctctaccggc     480
   accccgaagg gtgtggctgt ccctcaccgc tcggtggcag agctgaccgg taatccgggt     540
   tggggtgtgg agcctggtga ggcggttctg atgcacgcgc cgtacacgtt tgatgcaagc     600
   ttgtttgaga tttgggttcc gctggtgagc ggtgcgcgtg ttgtgattgc tgctccgggt     660
   gcggtcgacg cccgtcgctt gcgtgaagcg gtcgcagctg gcgtgacccg cgttcatttg     720
   acggcgggta gctttcgtgc cgtggccgaa gagagcccgg agagcttcgc gcacttccgc     780
   gaagttctga ccggtggcga tgtggtgccg gcctatgctg tccagaaagt tcgtgccgcg     840
   tgtccacatg ttcgtatccg ccatttgtat ggtccgaccg aaacgacgct gtgcgctacc     900
   tggcagctgc tggaaccggg cgacgtggtt ggcccggttc tgccgatcgg tcgcccgctg     960
   ccgggtcgtc gcgcatgggt tctggatgcg agcctgcgtc cggtcgagcc aggcgtcgtc    1020
   ggcgacctgt acctgtccgg tgcaggcctg gcggacggtt atctggaccg tgccggtctg    1080
   acggcggaac gtttcgttgc cgatccaagc gctgccggtc gtcgcatgta tcgcaccggt    1140
   gacctggcgc agtggaccgc ggacggcgag ctgctgtttg caggccgtgc cgatgatcaa    1200
   gtgaaggttc gtggcttccg tattgagccg ggtgaggttg aggcagcgct gaccgcgcag    1260
   ccgcacgtcc gcgaagcggt ggttgttgcg atcgacggtc gcctgatcgg ctacgtcgtg    1320
   gccgatggtg acgtggatcc ggtcctgatg cgtcgccgcc tggcggcaag cctgccggaa    1380
   tacatgattc ctgcggcact ggtgaccttg gacgcactgc cgctgacggg cagcggtaag    1440
   gttgaccgcc gtgcgttgcc ggagccggat tttgcgagcg ctgcccctcg tcgtgaaccg    1500
   ggcacggaac cggaggaccc agctttcttg tacaaagttg gcattataag aaagcattgc    1560
   ttatcaattt gttgcaacga acaggtcact atcagtcaaa ataaaatcat tatttgccat    1620
   ccagctgata tcccctatag tgagtcgtat tacatggtca tagctgtttc ctggcagctc    1680
   tggcccgtgt ctcaaaatct cggttctcgt agccaccatc atcaccatca ctgacctgca    1740
   gg                                                                   1742
<210> 11
   <211> 1730
   <212> DNA
   <213> Artificial Sequence
<220>
   <221> source
   <222> 1..1730
   <223> /organism="Artificial Sequence" /note="Synthetic DNA" /mol_type="unassigned DNA"
<400> 11
   catatgaact ccgtactgtc caccccaacc gtcccagagc tgttcgcgcg tcaggcagag      60
   cgcactccgg aagctgtggc agtcgttgat ggtgatcgct ttgtcaccta ccgtcaactg     120
   gacgagctgg caggccgtct ggcaggccgc ttgattggtc gtggtgttcg tcgcggcgac     180
   cgtgtcgcgg tcctgatgga acgttctgcg gatctggtcg tcaccctgct ggccgtttgg     240
   aaggctggcg ctgcgtacgt tccggttgat gcggcgcatc cggcaccgcg tgtggcattc     300
   gtggtggctg acagcggtgc gagcctgatg gcatgctcgg cagcgacggc cggtcgcgtg     360
   ccggagggcg ttgagccagt ggtcgtgact gatgaaggtc gtggcgacgc gagcgcggtt     420
   ccggtcagcc cgggtgatct ggcctacgtg atgtatacca gcggcagcac gggcacgccg     480
   aaaggtgtcg ctgttccgca tcgcagcgtt gcggagctgg cgggtaatcc aggttgggcg     540
   gttaaaccgg gcgatgcgat tctgatgcac gcgcctcacg cgtttgacgc cagcctgttc     600
   gagatctggg ttccgttggt tagcggtgcc cgcgttgtca tcgcggagcc aggcgctgtt     660
   gatgcccgtc gtctgcgcga agcgatcgca gcaggtgtta ccaaagttca cctgactgcc     720
   ggtagctttc gtgctctggc cgaagagagc agcgaaagct ttgccggcct gcaggaagtg     780
   ctgacgggtg gcgatgtggt gccggctcac gcagtcgaaa aggtccgtaa ggcagtgccg     840
   caagcgcgca ttcgtcacct gtatggcccg accgaaacca cgctgtgtgc cacctggcat     900
   ctgctgcagc cgagcgaggc gttgggtccg gtgctgccga ttggccgtcc gttgccgggt     960
   cgtcgtgccc aagtgctgga cgcaagcctg cgtccgctgc cgcctggcgt ggtgggcgat    1020
   ctgtatttga gcggtgcggg cctggcggac ggttacctgg atcgtgcggc cttgaccgca    1080
   gagcgcttcg tggccgatcc gtccgttccg ggtggccgta tgtaccgcac gggtgacctg    1140
   gtccagtgga cggctgacgg tgagctgctg tttgttggtc gtgcggacga ccaggtgaag    1200
   atccgtggtt tccgtatcga accgggtgaa atcgaagcag cactgacggc gcaaccggac    1260
   gttcatgagg cggttgtggt cgcgatcgac ggtcgcctga ttggttatgc agtgaccgac    1320
   gtggatccgg tggttttgcg cgagcgtttg ggcgcgaccc tgccggaata catggttcct    1380
   gcagtcgtta tcaccttgga tggcctgccg ctgacccgta atggcaaagt cgaccgtgcg    1440
   gcgctgccgg caccggtttt tggcaccaac gccgcaggtc gcgagccggc gaccgaggcg 1500
   gaggacccag ctttcttgta caaagttggc attataagaa agcattgctt atcaatttgt 1560
   tgcaacgaac aggtcactat cagtcaaaat aaaatcatta tttgccatcc agctgatatc 1620
   ccctatagtg agtcgtatta catggtcata gctgtttcct ggcagctctg gcccgtgtct 1680
   caaaatctcg gttctcgtag ccaccatcat caccatcact gacctgcagg 1730
<210> 12
   <211> 1667
   <212> DNA
   <213> Artificial Sequence
<220>
   <221> source
   <222> 1..1667
   <223> /organism="Artificial Sequence" /note="Synthetic DNA" /mol_type="unassigned DNA"
<400> 12
   catatgctgc cggtcggccg cttaggcgtt acttcagaac ctgcgagagc cagcgttgtt 60
   gagcgctgga atagcaccgg cgaagcggcg aatcgcacca gcgttttgga gctgttccgt 120
   caacaagctg atgcgtcccc ggacgcggtg gccgtgatgg atgcggctcg cacgctgtcg 180
   tatgctgacc tggatcgcga gagcgaccgt ctggcaggtt acctggcggc aatgggtgtc 240
   cgccgtggtg atcgtgtcgg tgttgttatg gagcgtggta cggatctgtt cgttgctctg 300
   ctggcagtgt ggaaagcagg cgcagcacag gtcccggtta acgttgatta tccggcggag 360
   cgtattgagc gtatgctggc ggatgcgggt gcgagcgttg cggtgtgtct ggaagccacc 420
   cgtaaagcag tgccggatgg tgtggagccg gttgtcatgg acgtcccggc catcgacggc 480
   gtccgccatg aggctccgca ggtgacggtt ggtgcacacg acctggccta cgtcatgtat 540
   acgagcggca gcacgggcgt gccgaagggt gtcgccgtgc cgcatggctc tgttgcggcc 600
   ctggcgagcg accctggttg gtcccaaggc ccggacgact gcgtcctgct gcacgcaagc 660
   cacgcctttg atgcttcctt ggtcgaaatc tgggtcccgc tggtcaatgg tagccgcgtc 720
   atggttgcgg aaccgggtgc ggtggatgcg gaacgtttgc gtgaagcgat cagccgtggt 780
   gtgacgaccg ttcacctgac ggcgggtgca ttccgtgcag tcgcagagga gagcccggac 840
   tccttcaccg gcctgcgcga gatcctgacc ggcggtgatg cggttccgtt ggcaagcgtc 900
   gttcgtatgc gtcgtgcttg cccggatgta cgtgttcgtc agttgtacgg tccgaccgaa 960
   attaccctgt gtgcaacctg gcacgtgatt gagccgggtg ccgaaacggg tgacaccctg 1020
   ccgattggtc gcccgctggc aggccgtcag gcgtatgtgc tggatgcgtt tctgcaacca 1080
   gttgcaccta acgtgacggg cgaattgtac attgctggtg cgggcctggc acatggctat 1140
   ctgggcaaca acggtagcac cagcgaacgt tttatcgcga acccgttcgc gtctggcgaa 1200
   cgcatgtacc gtaccggcga tttggcacgt tggaccgacc agggtgaact gctgttcgcc 1260
   ggtcgcgctg acagccaagt gaaaattcgc ggttaccgcg ttgagccagg cgagatcgaa 1320
   gtggcactga cggaggtgcc gcacgttgcc caggcggtcg tggtggcccg tgaggaccat 1380
   ccgggtgaca agcgcctgat cgcctacgtt actgccgagg aaggtccggc gctggcggca 1440
   gatgcggtac gtgagcatct ggcagcgcgt atgccggagt ttatggttcc ggcggtggtg 1500
   ctggtgctgg atagcttccc actgaccctg aatggtaaga ttgaccgtgc ggcgctgccg 1560
   gcaccagaat ttaccggcaa agcagcgggt cgtgagccgc gcaccgagac tgagcgtgtc 1620
   ttgtgcggta gccgttccca ccaccatcat caccactaac ctgcagg 1667
<210> 13
   <211> 1661
   <212> DNA
   <213> Artificial Sequence
<220>
   <221> source
   <222> 1..1661
   <223> /organism="Artificial Sequence" /note="Synthetic DNA" /mol_type="unassigned DNA"
<400> 13
   catatggtag gcagactggg cgtgacgagc gaaccgacga gagcagcggt ggtggagcgt      60
   tggaactcga ccggcgaggc ggctgccgaa acgagcgtgc tggaactgtt tcgtcgccag     120
   gcaggtgcga gcccggatgc agttgccgtc gtggcgggtg aacgtacgct gagctacgcg     180
   gatctggacc gtgagagcga tcgtctggcg ggtcatttgg caggcattgg cgttggtcgt     240
   ggtgatcgcg tcggtgtagt gatgacccgt ggtgcggact tgtttgtcgc actgctgggc     300
   gtttggaaag ccggtgcagc acaagtgcct gttaacgttg attacccggc tgagcgtatc     360
   gaacgtatgc tggctgatgt cggtgcaagc gtcgcggtgt gtgtagaggc gacccgcaaa     420
   gcagtgccgg atggtgttga gccggtcgtt gtcgatctgc cggttatcgg tggtgttcgt     480
   ccggaagccc cacctgtgac ggtgggtgcc cacgacgtcg cgtacgtcat gtacacgagc     540
   ggctccacgg gcgttccgaa ggcagtggcg gtcccacacg gttctgtggc ggcactggca     600
   agcgacccgg gttggagcca gggtccgggt gactgcgttc tgctgcacgc atctcatgcg     660
   tttgacgcat ctctggtgga gatttgggtt ccgctggtga gcggtgcccg cgttctggtg     720
   gcggagccgg gcacggtgga tgcggaacgc ctgcgtgaag cggttagccg cggtgtcacc     780
   accgtgcacc tgaccgcagg tgccttccgt gcggttgccg aagagagccc agatagcttc     840
   atcggtctgc gtgagatcct gacgggtggt gacgccgtcc cgctggcgag cgtggttcgc     900
   atgcgccaag cgtgcccgga cgttcgtgtc cgtcagctgt atggcccgac cgagatcacc     960
   ctgtgcgcca cctggctggt cctggaaccg ggtgcggcga ctggtgacgt cctgccgatt    1020
   ggccgtccgc tggcaggtcg ccaagcctat gtgttggatg ctttcctgca acctgttgcg    1080
   ccgaacgtca ccggcgaact gtacctggcg ggtgcaggcc tggctcacgg ttatctgggt    1140
   aatactgccg cgaccagcga gcgcttcgtt gcgaacccgt tttccggcgg tggccgtatg    1200
   tatcgtacgg gtgacctggc acgctggacc gaccagggcg agctggtgtt cgctggccgt    1260
   gcggatagcc aggttaagat ccgtggttac cgtgtcgaac cgggcgaagt tgaggtcgca    1320
   ctgaccgagg tgccgcatgt tgcgcaggca gtcgtggtgg cccgtgaggg ccaaccgggt    1380
   gagaaacgcc tgattgcgta tgtgaccgcg gaagcgggtt ccgcgttgga atctgcggcg    1440
   gttcgcgccc acctggccac ccgtctgccg gagttcatgg tcccgagcgt cgtggtcgtt    1500
   ttggagtcct tcccgttgac cctgaatggc aagattgacc gtgccgcttt gccagcgccg    1560
   gaatttgcgg gtaaagcagc gggtcgtgag ccgcgtaccg aagcagagcg tgttttgtgt    1620
   ggtagccgca gccatcatca tcaccaccac taacctgcag g                        1661
<210> 14
   <211> 1661
   <212> DNA
   <213> Artificial Sequence
<220>
   <221> source
   <222> 1..1661
   <223> /organism="Artificial Sequence" /note="Synthetic DNA" /mol_type="unassigned DNA"
<400> 14
   catatgagca cggtagcaga cgtagacgta accagcgcag cagaacgcgc gctggtagtg      60
   gatgaatggg gtgcagcggc ggaggcggca ccgagccgcc tggcactgga actgttcgac     120
   ggccaagtgg agagccgtcg cgatgccatc gcggtcgttg accgcgacca ggccatgagc     180
   tatggcgttc tggcggagga tgccgagcgt ctggccggct atttgaatgg tcgtggcgtt     240
   cgtcgcggtg atcgtgtcgc ggttgttgtg gagcgctctc atgacctgat tgccaccctg     300
   ctggcggtct ggaaggcagg cgcagcctat gtcccggtag atccggcata cccgctggaa     360
   cgtgtcaagt tcatgctggc agacgcggac ccggcagctg tcgtctgtac cgcaggctat     420
   cgtgacagcg tcctggacgg tggcttggac cctatcgttt tggatgatcc gcaaacccgt     480
   caggcggtca gcgaatgttc tcgtttgtcc gtgggcacca ccgccgacga cgttgcgtat     540
   gtcatgtaca cgagcggtag caccggcacc ccgaaaggcg tcgccgtcag ccacggtaac     600
   gttgcagcgc tggtgggtga gccgggttgg cgtgttggcc cggatgacgc agttctgatg     660
   cacgcaagcc acgccttcga catcagcctg tttgaaatgt gggttcctct ggtgtccggt     720
   gctcgcgtgg tgctggctgg ttccggtgcg gtggacggtg cggcgctggc ggcgtatgtg 780
   gctgatggcg tgaccgcagc gcatctgacg gcaggcgctt tccgtgttct ggctgaggag 840
   agcccggagt ccgttgcggg tctgcgtgaa gttttgaccg gcggtgatgc ggttccactg 900
   gcagcggttg aacgtgttcg tcgtacctgc ccggacgtgc gcgtgcgtca cctgtacggc 960
   ccgacggagg caaccctgtg cgcgacgtgg ctgctgttgg aaccgggcga tgaaacgggt 1020
   ccggttttgc caatcggccg tccgctggcg ggtcgccgcg tctacgtgct ggatggtttc 1080
   ctgcgtccgg ttccaccggg tgtggctggt gagctgtacg tagccggtgc aggtgtcgct 1140
   caaggctacc tggaacgtcc ggcgttgact gcggagcgtt ttgtcgccga tccgtttgtg 1200
   gcccacggcc gtatgtaccg tactggtgat ctggcgtact ggacgggtaa aggtgctctg 1260
   gcatttgcgg gtcgtgcaga tgatcaggtg aaaattcgtg gctaccgcgt ggagccgggt 1320
   gaaattgagg tggttctggc cggtctgccg ggtgttggcc aggcggtcgt gctggcccgt 1380
   gatgaacacc tgattggcta tgcagtggct gaggctggtc atgagctgga cccggtgcgc 1440
   ctgcgtgagc agctggcgga caccctgccg gagttcatgg tcccggcagc ggtcctggtt 1500
   ttgggcgaac tgccgctgac ggtcaacggt aaggttgatc gccaagcgtt gccaggtcca 1560
   gactttgcaa gcaaagcagc gggtcgcgct ccggcgaccg acgcggagcg cgtgctgtgc 1620
   ggttctcgta gccaccatca tcaccatcac taacctgcag g 1661
<210> 15
   <211> 1652
   <212> DNA
   <213> Artificial Sequence
<220>
   <221> source
   <222> 1..1652
   <223> /organism="Artificial Sequence" /note="Synthetic DNA" /mol_type="unassigned DNA"
<400> 15
   catatgctca ccgtagccgc catcgacgtc acctcagccg ccgaacgcga ccgtgtcgcg      60
   cgttggggtg cggctgtcgg tgctcgcccg gaccgtctgg cgctggacct gttcgcccgt     120
   caagttgctc aacgcccgga cgaggtggcc gttgcagacg gcgaccgcgt catgagcttc     180
   ggcgaactgg cggagcgtgc ggatcgtttg gcgggtcatc tgagcgcacg cggcgttcgt     240
   cgtggcgatc gcgtggcggt tgtcatggag cgctcgggcg aactgattgc gaccctgctg     300
   gcggtgtggc gcgcaggcgc agcgtttgtg ccggttgatc cggcataccc tgcggagcgc     360
   gttaagtttt tgctgaccga cgctgagccg gtggcggcag tgtgcaccgc tgcatttcgt     420
   gcggcggtcc tggatggcgg tctggaggcc attgtcgtag atgatccggg tacgtggccg     480
   gctgtcgcgc cgtgtcctcc ggtgccgact ggtccagatg acctggcata cgtgatgtat     540
   accagcggct ccacgggcac cccgaaaggt gtggctgtta gccacggtga tgttgcggcg     600
   ttggttggcg atccgggctg gcgcacgggt ccgggtgaca ccgtgctgat gcacgcttct     660
   cacgcattcg acatttcctt gttcgaaatc tgggtcccgc tgctgagcgg tgcgcgtgtg     720
   atgatcgccg gtccaggtgc agtcgatggt gccgcgctgg ccgctcaggt tgcagcaggt     780
   gtcaccgctg cgcatctgac cgctggcgca ttccgtgttc tggcggaaga aagcccggag     840
   agcgtcgcgg gtctgcgtga ggtgctgacg ggtggcgacg cagttccgct ggcagcagtg     900
   gagcgcgtgc gccgtgcctg cccggacgtt cgtgttcgtc acctgtatgg cccgaccgaa     960
   accacgctgt gtgcaacgtg gtggttgctg gaaccgggtg atgaaacggg tccagtgctg    1020
   ccgatcggtc gtccgctggc cggtcgccgc gtgtatgtgc tggacgcatt cctgcgtccg    1080
   ctgccgccag gcaccaccgg cgagctgtat gttgcgggtg cgggtgttgc acagggctac    1140
   ttgggtcgtc cggcgctgac ggcggaacgc tttgttgcgg acccttttgc gcctggtggc    1200
   cgtatgtacc gcactggtga tttggcctac tggaccgagc agggtactct ggcgtttgcg    1260
   ggtcgtgcgg acgatcaagt gaaaattcgt ggttatcgtg ttgagccggg tgaagtggag    1320
   gcggtgctgg gcggcttgcc gggtgtcgca caggccgtag tatgcgtccg tggtgagcat    1380
   ctgattggtt acgtggttgc cgaagccggt cgcgatctgg acccggagcg tctgcgtgcg    1440
   cgtttggcag ccaccctgcc ggagttcatg gtgccagcgg ctgtgctggt cctggcagat    1500
   ttgccgctga cggttaacgg taaggtcgat cgtccggctc tgccggaacc ggacttcgcc    1560
   gctaaaagca cgggccgtgc accggccacg gctgcggaac gcatcctgtg tggcagccgt    1620
   agccatcacc accaccatca ctaacctgca gg                                 1652
<210> 16
   <211> 1661
   <212> DNA
   <213> Artificial Sequence
<220>
   <221> source
   <222> 1..1661
   <223> /organism="Artificial Sequence" /note="Synthetic DNA" /mol_type="unassigned DNA"
<400> 16
   catatgcttc cagtcggtcg cttaggcgta acctcagatg caactcgcac gagcgaagtc      60
   gagcgctgga atgcgacggg cgaagctgcg ggtggtgcga gcgtggttga gctgttccgt     120
   cgtcgttctg cgggcacccc ggatgccgtt gcggtcgtgg acggtgatcg caccctgagc     180
   tacggcgacc tggaccgcga gtcggatcgt ctggccggtc gcttggcaga aacgggtgtg     240
   cgtcgcggcg atcacgtggg tgtcgtcctg gaacgcggtg cggacctgtt cgtagccttc     300
   ctggcggttt ggaaggcggg tgctgcttac gttccagttc acgtggatta tccgccggtc 360
   cgtattgaac gtatgctggc ggatgccggt gtgacggtcg cggtttgtgc ggaaggtacg 420
   cgcaacgccg tgccggacgg cctggagccg gttccggttg atgcaccgtg ggcgggtgaa 480
   acccgccacg aaaccccgac ggtgacggct cgtgacgcgg cctacgttat gtacaccagc 540
   ggcagcaccg gcgagccgaa aggcatcgtt gttccgcatg gcagcgttgc cgcactggca 600
   ggtgacccag gttgggctct ggacgctgac gattgcgtgc tgatgcacgc gagccatgcg 660
   ttcgatgctt ccttgtttga aatttgggca ccgctggtcc gtggcgcacg tgtcatggtc 720
   gcggagcctg gtgcggtgga tacccagcgt ctgcgtgaag cggtggcgcg tggtgtcacc 780
   accgtgcacc tgaccgccgg tagcttccgc gtcctggcgg aggagtctcc gggttctttt 840
   gatggtctgc gcgagatcct gactggtggc gacgtggtgc cgctggcaag cgtcgcacaa 900
   ttgcgtcgcg cctgcccgga tgtgcgcgtc cgtcacctgt atggcccgac ggaaaccacc 960
   ctgtgcggca cctggcacct gctggagcct ggcgacgaac cgggtgacgt gctgccgatc 1020
   ggtcgtccgc tggcaggccg tcgtgcgtat gtgctggacg catttctgca accagtggcg 1080
   ccgaatgtta ctggcgagct gtatctggcg ggtgtgggtt tggcgctggg ttacttgggt 1140
   gcccgtggtg cgaccagcga gcgttttgtt gcagacccgt tcgttcctgg tgagcgtatg 1200
   taccgtactg gcgatctggc gcgtcgcaac gatcgcggtg aattgctgtt tgcaggccgt 1260
   gcagacgcgc aggttaagat tcgtggttat cgtgtcgagc cgacggagat cgaaaccgta 1320
   ttggcagaag caccgcaagt ggcacagacg gtcgttgttg cccgcgagga cggtccgggt 1380
   gagaagcgtc tgattgcata cgcgattgcg gaaccggacc aggttctgga cccggaggcc 1440
   ttgcgtgaac atctggcagc gcgtttgccg gagtttatgg ttccggcagc tgtggttgtg 1500
   ctggatgact tcccgctgac catcaacggc aaaatcgacc gtgaagcgct gccggcaccg 1560
   gagttcagcg caaaacctgc tggccgtgag ccgcgtaccg aggcggagcg tgttctgtgt 1620
   ggttcccgca gccatcatca ccaccaccat taacctgcag g 1661
<210> 17
   <211> 1613
   <212> DNA
   <213> Artificial Sequence
<220>
   <221> source
   <222> 1..1613
   <223> /organism="Artificial Sequence" /note="Synthetic DNA" /mol_type="unassigned DNA"
<400> 17
   catatggtac tggtaggcag agtaggctta gtgggcagac tcgaacgtgg tctggtcgtc 60
   gaaggttgga acgccaccgc cggtgacgtg ccgtctggca gctctgtctt ggagatgttt 120
   cgtgcgcgtg tggcgcaagc accggaggcc gttgcggttg ttgacggtga gcgccaggtg 180
   agctacggcg agctggacgc ggacagcaac cgtatggcag cgtacctgca aggtcgtggt 240
   gtgggtcgtg gcgaccgcgt tgcagtccgc ttggagcgtt ctatcgacct gattgcagcg 300
   ttgttgggtg tgtggaaggc gggtgccgcg tatgtgccgg tggatagcgc gtatccggcc 360
   gaacgtgtgg cgttcatggt cgaagatagc gcaccagtgc tgacgatcga tgatccgtcg 420
   gttgtcaccg cagagggtga gccggaggtc gtggaaaccg caggtggtga cattgcttac 480
   gtgatgtaca cgagcggcag caccggcacg ccgaaaggcg tggccgttcc gcacgcatcg 540
   gtggccgcgt tggtcggtga accaggttgg ggtgttggtc cgggtgacgc agtgctgttc 600
   catgcgccac acgcctttga catctctctg tttgaagttt gggtcccgct ggcgagcggt 660
   ggccgtatcg ttgtcgcaga gccgagcatg gcggtggacg gtgcggccgt tcgtcgtcat 720
   atcgcggacg gtgtgaccca cgtccacgta acggcgggtc tgttccgtgt gctggcagaa 780
   gaggcaagcg attgtttcga tggtgtccat gaggtcctga ctggtggtga cgtcgttccg 840
   ctggaagcgg tggagcgcgt tcgcgctgcg tgcccagatg tgcgcgttcg ccacctgtat 900
   ggcccgactg aggtttcttt gtgcgctacc tggcacttgt tcgaaccggg tgaagaacag 960
   ggcgaggtcc tgccgctggg tcgtccgctg aacaatcgtc aagtttatgt tctggacccg 1020
   tttctgcaac cggttcctcc gggcgttacg ggtgagctgt acgttgcggg tgcaggtctg 1080
   gcgcgtggct acctgggtcg tgccggtctg tcggcggaac gcttcgtggc atccccgttt 1140
   gcagacggcg aacgtatgta tcgtaccggc gacctggtgc gttggaccac tggtgtcgag 1200
   ctggtgttcg tgggtcgcgc agacgcgcaa gtgaaaattc gtggtttccg cgttgagttg 1260
   ggtgaggtcg aagcggcact ggctgcccag cctgcggtgg cccaggcagt ggttgttgcg 1320
   cgcgaggacc gtccgggcga gaagcgtctg gtgggctacc tggtgccatc tggtgaagaa 1380
   ccggacactg aagcagttca cgcaagcctg gcagatcgtt tgccggaata catggttccg 1440
   gctgcgctgg tggtgctgga cgcgctgccg ctgacggtta atggtaaggt ggaccataag 1500
   gcgctgccgg ccccggaatt taccgcaacg gccagccgtg aaccgcgtac tgccgctgaa 1560
   aagctgctgt gcggcagccg tagccaccac catcatcacc actaacctgc agg 1613
<210> 18
   <211> 69
   <212> PRT
   <213> Actinoplanes teichomyceticus
<400> 18
   Met Thr Asn Pro Phe Asp Asn Glu Asp Gly Ser Phe Leu Val Leu Val
   1               5                   10                  15
   Asn Gly Glu Gly Gln His Ser Leu Trp Pro Ala Phe Ala Glu Val Pro
               20                  25                  30
   Asp Gly Trp Thr Gly Val His Gly Pro Ala Ser Arg Gln Asp Cys Leu
           35                  40                  45
   Gly Tyr Val Glu Gln Asn Trp Thr Asp Leu Arg Pro Lys Ser Leu Ile
       50                  55                  60
   Ser Gln Ile Ser Asp
   65
<210> 19
   <211> 69
   <212> PRT
   <213> Actinoplanes teichomyceticus
<400> 19
   Met Thr Asn Pro Phe Asp Asn Glu Asp Gly Ser Phe Leu Val Leu Val
   1               5                   10                  15
   Asn Gly Glu Gly Gln His Ser Leu Trp Pro Ala Phe Ala Glu Val Pro
               20                  25                  30
   Asp Gly Trp Thr Gly Val His Gly Pro Ala Ser Arg Gln Asp Cys Leu
           35                  40                  45
   Gly Tyr Val Glu Gln Asn Trp Thr Asp Leu Arg Pro Arg Ser Leu Val
       50                  55                  60
   Glu Gln Ala Asp Ala
   65
<210> 20
   <211> 69
   <212> PRT
   <213> uncultured soil bacterium
<400> 20
   Met Thr Asn Pro Phe Asp Asn Glu Asp Gly Thr Phe Phe Val Leu Val
   1               5                   10                  15
   Asn Asp Glu Gly Gln His Ser Leu Trp Pro Thr Phe Ala Glu Val Pro
               20                  25                  30
   Ala Gly Trp Thr Arg Val His Gly Glu Ala Thr Arg Gln Glu Cys Leu
           35                  40                  45
   Ala Tyr Val Glu Glu Asn Trp Thr Asp Leu Arg Pro Lys Ser Leu Ile
       50                  55                  60
   Gln Ala Leu Gly Ala
   65
<210> 21
   <211> 238
   <212> DNA
   <213> Artificial Sequence
<220>
   <221> source
   <222> 1..238
   <223> /organism="Artificial Sequence" /note="Synthetic DNA" /mol_type="unassigned DNA"
<400> 21
   ggatccagga ggaattacat atgaccaatc cgttcgacaa cgaggacggt tccttcctcg      60
   tgctcgtcaa cggcgagggc cagcattcgc tgtggccggc tttcgccgag gtcccggacg     120
   gctggacggg ggtccacggt ccggcctccc ggcaggattg tctcggctac gtcgagcaga     180
   actggacgga cctgcggccc aagagtctga tctcgcagat cagcgactga cctgcagg       238
<210> 22
   <211> 238
   <212> DNA
   <213> Artificial Sequence
<220>
   <221> source
   <222> 1..238
   <223> /organism="Artificial Sequence" /note="Synthetic DNA" /mol_type="unassigned DNA"
<400> 22
   ggatccagga ggaattacat atgaccaatc cgttcgacaa cgaggacggt tccttcctcg      60
   tgctcgtcaa cggcgagggc cagcattcgc tgtggccggc tttcgccgag gtcccggacg     120
   gctggacggg ggtccacggt ccggcctccc ggcaggattg tctcggctac gtcgagcaga     180
   actggacgga cctgcggccc aggagcctgg tcgagcaggc cgacgcgtga cctgcagg       238
<210> 23
   <211> 238
   <212> DNA
   <213> Artificial Sequence
<220>
   <221> source
   <222> 1..238
   <223> /organism="Artificial Sequence" /note="Synthetic DNA" /mol_type="unassigned DNA"
<400> 23
   ggatccagga ggaattacat atgaccaacc cgttcgacaa cgaggacggc accttcttcg      60
   tgctggtcaa cgacgagggc cagcactccc tctggccgac cttcgccgag gtgcctgccg     120
   gctggacccg cgtgcacggt gaagccaccc ggcaggagtg cctcgcgtat gtcgaggaga     180
   actggacgga cctgcggccg aagagcctca tccaggccct cggcgcctga cctgcagg       238
<210> 24
   <211> 1742
   <212> DNA
   <213> Artificial Sequence
<220>
   <221> source
   <222> 1..1742
   <223> /organism="Artificial Sequence" /note="Synthetic DNA" /mol_type="unassigned DNA"
<400> 24
   catatgaact ccgcagcgca ggccacatcg acggtgccgg agctgctcgc ccggcaggtg      60
   acccgggccc ccgatgcggt ggccgtggtg gaccgggacc gggttctgac gtaccgggaa     120
   ctcgatgagc tcgcgggccg gttgtccgga cgtctgatcg gccggggcgt ccgccgcggg      180
   gaccgcgtgg cggtcctgct ggaccgttcg gcggacctgg tggtgacgct gctcgcgatc      240
   tggaaggccg gggcggcgta tgtgccggtc gatgccggct atcccgcgcc gcgtgtggcg      300
   ttcatggtgg cggactcggg agcctcccgc atggtgtgct cggccgcgac gcgtgacggc      360
   gtaccggagg ggatcgaggc gatcgtcgtc acggatgagg aggcgttcga ggcctcggcg      420
   gccggggcgc gaccgggaga tctggcgtac gtgatgtaca cctccggctc gaccggcatc      480
   ccgaagggcg tggcggttcc gcatcgcagc gtcgcggagc tggccgggaa tcccggctgg      540
   gcggtggagc cgggcgacgc ggtcctgatg cacgcgccgt acgccttcga cgcgtcgctg      600
   ttcgagatct gggtgccgct ggtttccggg ggccgggtgg tgatcgccga gccggggccg      660
   gtggacgccc ggcgcctgcg ggaggcgatc agctccgggg tgaccagggc gcatctgacc      720
   gccggcagct tccgcgcggt ggcggaggag tcgccggagt ccttcgccgg gctgcgcgag      780
   gtgctgaccg gcggtgacgt ggtgccggca cacgccgtgg cgcgggtccg ctcggcctgt      840
   ccccgggtgc ggatccggca cctgtacggc ccgacggaga cgacgctgtg cgccacatgg      900
   catcttctgg agccggggga cgagatcggc ccggtgttgc cgatcggccg tccgctcccg      960
   ggccggcgcg ctcaggtgct cgacgcgtcg ctgcgggccg tggcgccggg cgtgatcggt     1020
   gacctgtacc tgtccggcgc cggtctggct gacggctacc tgcgccgggc agggctgaca     1080
   gcggagcgat tcgtggccga cccgtccgcg cccggggcga ggatgtaccg caccggcgac     1140
   ctcgcgcagt ggaccgccga cggtgcgttg ctgttcgcgg gccgggccga cgaccaggtg     1200
   aaggttcgcg gcttccggat cgagccggcc gaggtcgagg ccgcgttgac cgcgcagccg     1260
   ggcgtccacg aggccgtggt ccgagcggtc gacgggcgcc tggtcggcta tgtggtggcg     1320
   gagggggacg cggaaccggc tgtcctgcgc gagcgtgtcg gtgcggtgct gccggagtac     1380
   atggtcccgg ccgcggtgat cacactggac gcgctgccgc tgaccggcaa cggcaaggtg     1440
   gaccgggcgg ctctgccggc tccggtcttc gcggcggacg ctccggggcg cgaacccggc     1500
   accgaggcgg agcgcgtgct gtgcgggctg ctgtccgagg tgctcggcct gaaccgggtc     1560
   ggagtcgacg agagcttctt cgagctgggc ggagactcca tcgcggcgat ccggctggcg     1620
   gcgcgtgcgt cccgggcggg cctgctcgtg acgcccgccc agatcttcaa ggagaggact     1680
   gtcgcacggc tggcggccgt gggttctcgt agccaccatc atcaccatca ctgacctgca     1740
   gg                                                                    1742
<210> 25
   <211> 3164
   <212> DNA
   <213> Artificial Sequence
<220>
   <221> source
   <222> 1..3164
   <223> /organism="Artificial Sequence" /note="Synthetic DNA" /mol_type="unassigned DNA"
<400> 25
   catatgagca ccgttgcgga tgtggacgtt accagcgcag ccgaacgtgc cctggtagtg        60
   gatgagtggg gcgctgctgc ggaggcagcg cctagccgcc tggcactgga gctgtttgac       120
   ggtcaagtgg agagccgtcg tgacgcgatt gcggtcgttg atcgtgatca ggcaatgagc       180
   tacggcgttc tggccgaaga tgcagaacgc ctggcgggct acctgaatgg ccgtggtgtt       240
   cgtcgtggtg atcgcgtggc agtcgtggtg gaacgtagcc atgacttgat tgccactctg       300
   ttggccgttt ggaaagctgg cgcagcctac gtgccggttg acccggcata cccgctggag       360
   cgcgtgaaat tcatgctggc cgatgccgac ccggcagcag tggtttgtac ggcaggctat       420
   cgtgactccg tcttggatgg tggtttggac ccgattgttc tggacgatcc gcagacgcgc       480
   caagcagtca gcgaatgcag ccgtctgtct gtaggcacta ctgcggacga tgtagcttac       540
   gtcatgtaca cctctggttc gaccggcacc ccgaaaggcg tcgcagtttc ccacggcaac       600
   gtcgcggctc tggttggcga accgggctgg cgcgtcggtc cggacgatgc cgttctgatg       660
   cacgcaagcc atgcctttga tattagcctg ttcgaaatgt gggtgcctct ggttagcggt       720
   gctcgcgtgg ttttggctgg tagcggcgcc gttgatggtg cggcactggc ggcatacgtc       780
   gcagacggcg tgaccgcagc ccatctgacg gcaggcgcgt tccgtgtcct ggcagaagag       840
   agccctgaga gcgtcgcggg tttgcgtgag gtgttgactg gcggtgacgc cgtgccgctg       900
   gccgcagttg agcgcgttcg tcgtacctgc ccggatgttc gtgtgcgtca cctgtatggt       960
   ccgaccgagg cgacgctgtg tgcaacgtgg ttgctgctgg aaccgggtga tgaaacgggt      1020
   cctgttctgc caatcggtcg tccgctggcg ggccgtcgtg tttatgtact ggatggtttc      1080
   ctgcgtccgg tgcctccggg cgttgcaggc gagctgtacg ttgcgggtgc gggtgttgca      1140
   caaggttatc tggagcgccc tgcactgacg gcggagcgtt ttgttgcaga tccgtttgtt      1200
   gcgcacggtc gtatgtaccg cacgggtgac ctggcatact ggacgggtaa gggtgcactg      1260
   gcatttgcag gtcgcgcaga tgaccaggtg aagatccgtg gttaccgtgt cgagccgggt      1320
   gaaattgaag ttgtcctggc gggtctgccg ggtgtcggtc aagcggttgt gttggcgcgt      1380
   gacgagcatc tgatcggcta cgcagttgcg gaggccggtc atgaactgga cccggtgcgc      1440
   ctgcgcgaac agctggcgga caccctgccg gagttcatgg ttccggctgc cgtcctggtc      1500
   ctgggtgagc tgccgctgac ggtgaacggt aaagtggatc gtcaggcatt gccgggtccg      1560
   gacttcgcaa gcaaagcggc aggccgtgct ccggcaaccg acgcagaacg tgtgctgtgt      1620
   ggtgtttttg ccgaggtgct gggcttggat cgcgtttcgg tcgaagatag ctttttcgaa      1680
   ttgggtggcg atagcatcag cagcatgcaa gttgccgcac gtgctcgtcg tgagggtatt 1740
   tctttgaccc cgcgtcaggt gttcgagtat cgtaccccgg aacgtctggc agcgctggct 1800
   caagaagccc aaccgacccg tcgtgcggag gtaagcggtg tgggtgagat tccgctgacc 1860
   cctgttatgc gtgctctggg cgatgacgct gtgcgcccga attttgccca agcacgtgtc 1920
   gtcggtacgc cggcaggcct gaaccaagat agcctggtga aagcgctgca agctgtgctg 1980
   gatgttcacg acctgctgcg cgctcgcgtc cagagcgacg gtcgcttgat tgtcgcagag 2040
   ccaggtgccg tgaatgcagc aggcttggtg actcgtgtgg cagccgagag cggtaacctg 2100
   gatgagattg cggaaggtca agtttctgcg gcgatgggca ccctgaaccc gagcgcaggt 2160
   atcatggctc gtgttgtttg gatcgatgcg ggctccgatg aaccaggccg tctggctttt 2220
   gtggcccacc acctggcagt ggatgccgtt agctggggca tcttgctgcc ggatctgcgt 2280
   agcgcgtatg acgcggtgat cgcaggtgaa accccagcat tggaaccggc agttacgagc 2340
   taccgtcagt gggcgctgcg tctggcggag caagcccgta gcgactccac ggtggctgag 2400
   gttgaccaat gggttgaact gttggacggc gcagaaagcg ttctggaaca gcaaacgggt 2460
   cagagccaca gctggagcga tgcgctgtcc ggccctgttg cccgtaccct ggtgtcccag 2520
   ttgccggctg cgttccactg cggcattcag gatgttctgc tggcaggttt ggccggtgcg 2580
   gtggcgcgtg tgcgcggtgc cggtgctggt ttgctggttg atgttgaggg tcacggtcgt 2640
   gatgccgccg acggtgagga cctgttgcgc accgttggtt ggttcaccag cgtgcacccg 2700
   gtccgcttgg atttggcgga tctgagcttg aaagctgtca aagaacaggt ccgtgcggtt 2760
   cctggcgatg gcttgggtta tggtctgctg cgctatctga atccggaaac cgctgcgcgt 2820
   ctggccggtc tgccgagcgc tcagattggt ttcaactatc tgggccgcac ctccctgacc 2880
   ctgaaaaatc cggcttggga ggtgagcggc gagggtccac tgggcggtgg cccggacacc 2940
   gccctggccc acctggttga agtcggtgct gaagtccaag ataccccgga tggtccgcgt 3000
   ctgggtctgg ccattgatgg ccgcgacatt gatccggcga cggtccagca gctgggtgaa 3060
   gcgtggctgg agatcctgac cgccttggcg gatgacgccg gtgcaggtgg ccacacagag 3120
   accggttctc gtagccacca tcatcaccat cactaacctg cagg 3164
<210> 26
   <211> 538
   <212> PRT
   <213> Amycolatopsis balhimycina
<400> 26
   Leu Thr Val Ala Gly Val Glu Val Thr Thr Ala Ala Glu Arg Ala Leu
   1               5                   10                  15
   Val Ala Gly Glu Trp Gly Ala Ser Thr Ser Ala Pro Pro Ser Leu Pro
               20                  25                  30
   Ala Leu Asp Leu Phe Gly His Gln Val Ala His Arg Arg Asp Glu Pro
           35                  40                  45
   Ala Val Val Asp Gly Asp Arg Thr Val Ser Tyr Gly Glu Leu Ala Glu
       50                  55                  60
   Arg Ala Glu Arg Leu Ala Gly Tyr Leu Asn Gly Arg Gly Val Arg Arg
   65                  70                  75                  80
   Gly Asp Arg Val Ala Val Val Leu Asp Arg Ser Pro Asp Leu Ile Ala
                   85                  90                  95
   Thr Leu Leu Ala Val Trp Lys Ala Gly Ala Ala Tyr Val Pro Val Asp
               100                 105                 110
   Pro Ala Tyr Pro Val Glu Arg Arg Lys Phe Met Leu Ala Asp Ser Gly
           115                 120                 125
   Pro Ala Ala Val Val Cys Ala Glu Ala Tyr Arg Ala Ala Val Pro Asp
       130                 135                 140
   Thr Cys Pro Glu Pro Ile Val Leu Asp Asp Pro Arg Thr Arg Gln Ala
   145                 150                 155                 160
   Val Ala Glu Ser Pro Arg Leu Ser Ala Gly Thr Ser Ala Asp Asp Leu
                   165                 170                 175
   Ala Tyr Val Met Tyr Thr Ser Gly Ser Thr Gly Thr Pro Lys Gly Val
               180                 185                 190
   Ala Val Ser His Gly Asn Val Ala Ala Leu Ala Gly Glu Pro Gly Trp
           195                 200                 205
   Arg Val Gly Pro Gly Asp Ala Val Leu Leu His Ala Ser His Ala Phe
       210                 215                 220
   Asp Ile Ser Leu Phe Glu Met Trp Val Pro Leu Leu Ser Gly Ala Arg
   225                 230                 235                 240
   Val Val Leu Ala Gly Pro Gly Ala Val Asp Gly Ala Ala Leu Ala Ala
                   245                 250                 255
   Tyr Val Ala Gly Gly Val Thr Ala Ala His Leu Thr Ala Gly Ala Phe
               260                 265                 270
   Arg Val Leu Ala Asp Glu Ser Pro Glu Ala Val Ala Gly Leu Arg Glu
           275                 280                 285
   Val Leu Thr Gly Gly Asp Ala Val Pro Leu Ala Ala Val Glu Arg Val
       290                 295                 300
   Arg Gly Arg Val Arg Asn Val Arg Val Arg His Leu Tyr Gly Pro Thr
   305                 310                 315                 320
   Glu Ala Thr Leu Cys Ala Thr Trp Trp Leu Leu Glu Pro Gly Asp Glu
                   325                 330                 335
   Thr Gly Ser Val Leu Pro Ile Gly Arg Pro Leu Ala Gly Arg Arg Val
               340                 345                 350
   His Val Leu Asp Ala Phe Leu Arg Pro Val Pro Pro Gly Val Ala Gly
           355                 360                 365
   Glu Leu Tyr Val Ala Gly Ala Gly Val Ala Gln Gly Tyr Ser Ser Arg
       370                 375                 380
   Pro Ala Leu Thr Ala Glu Arg Phe Val Ala Asp Pro Ser Gly Ser Gly
   385                 390                 395                 400
   Ala Arg Met Tyr Arg Thr Gly Asp Leu Ala Tyr Trp Thr Glu Gln Gly
                   405                 410                 415
   Ala Leu Ala Phe Ala Gly Arg Ala Asp Asp Gln Val Lys Ile Arg Gly
               420                 425                 430
   Tyr Arg Val Glu Pro Gly Glu Ile Glu Val Val Leu Ala Gly Leu Pro
           435                 440                 445
   Gly Val Gly Gln Ala Val Val Thr Pro Arg Gly Glu His Leu Ile Gly
       450                 455                 460
   Tyr Val Val Ala Glu Ala Gly His Asp Ala Asp Pro Val Arg Leu Arg
   465                 470                 475                 480
   Glu Gln Leu Ala Gly Thr Leu Pro Glu Phe Met Val Pro Ala Ala Val
                   485                 490                 495
   Leu Val Leu Asp Glu Leu Pro Leu Thr Val Asn Gly Lys Val Asp Arg
               500                 505                 510
   Arg Ala Leu Pro Glu Pro Asp Phe Ala Ala Lys Ser Ala Gly Arg Glu
           515                 520                 525
   Pro Val Thr Glu Ala Glu Arg Val Leu Cys
       530                 535
<210> 27
   <211> 538
   <212> PRT
   <213> uncultured soil bacterium
<400> 27
   Leu Arg Val Ala Asp Val Asp Val Thr Ser Ala Ala Glu Arg Glu Leu
   1               5                   10                  15
   Val Val Asn Glu Trp Ser Ala Ala Ser His Ala Ala Pro Ser Arg Leu
               20                  25                  30
   Ala Pro Asp Leu Phe Gly Arg Gln Val Glu Arg Arg Arg Asp Glu Val
           35                  40                  45
   Ala Val Val Asp Gly Asp Arg Ala Met Ser Tyr Gly Glu Leu Ala Glu
       50                  55                  60
   Arg Ala Glu Lys Leu Ala Gly Tyr Leu Ser Gly Arg Gly Val Arg Arg
   65                  70                  75                  80
   Gly Asp Arg Val Ala Val Val Met Asp Arg Ser Pro Asp Leu Ile Ala
                   85                  90                  95
   Thr Leu Leu Ala Val Trp Lys Ala Gly Ala Ala Tyr Val Pro Val Asp
               100                 105                 110
   Pro Ala Tyr Pro Val Glu Arg Val Lys Phe Met Leu Ala Asp Ala Glu
           115                 120                 125
   Pro Ala Ala Val Val Cys Ala Glu Ala Tyr Arg Asp Ala Ala Leu Asp
       130                 135                 140
   Gly Gly Leu Asp Pro Ile Val Leu Asp Asp Pro Arg Thr Arg Gln Ala
   145                 150                 155                 160
   Val Ala Glu Cys Thr Arg Leu Ser Val Gly Ala Thr Ala Asp Asp Leu
                   165                 170                 175
   Ala Tyr Val Met Tyr Thr Ser Gly Ser Thr Gly Thr Pro Lys Gly Val
               180                 185                 190
   Ala Val Ser His Gly Asn Val Ala Ala Leu Val Gly Glu Pro Gly Trp
           195                 200                 205
   Ala Gly Ser Pro Asp Asp Ala Val Leu Met His Ala Ser His Ala Phe
       210                 215                 220
   Asp Ile Ser Leu Phe Glu Met Trp Val Pro Leu Leu Ser Gly Ala Arg
   225                 230                 235                 240
   Val Val Leu Ala Gly Ser Gly Ala Val Asp Gly Glu Ala Leu Ala Gly
                   245                 250                 255
   Tyr Val Ala Gly Gly Val Thr Ala Ala His Leu Thr Ala Gly Thr Phe
               260                 265                 270
   Arg Val Val Ala Glu Glu Ser Pro Glu Ser Ile Ala Gly Leu Arg Glu
           275                 280                 285
   Val Leu Thr Gly Gly Asp Ala Val Pro Pro Ala Ala Val Glu Arg Val
       290                 295                 300
   Arg Arg Thr Cys Pro Gly Val Arg Val Arg His Leu Tyr Gly Pro Thr
   305                 310                 315                 320
   Glu Ala Thr Leu Cys Ala Thr Trp Trp Leu Leu Glu Pro Gly Asp Glu
                   325                 330                 335
   Thr Gly Ser Val Leu Pro Ile Gly Arg Pro Leu Ser Gly Arg Arg Val
               340                 345                 350
   Tyr Val Leu Asp Ala Phe Leu Arg Pro Val Pro Pro Gly Val Ala Gly
           355                 360                 365
   Glu Leu Tyr Val Ala Gly Ala Gly Val Ala Gln Gly Tyr Leu Gly Arg
       370                 375                 380
   Ser Ala Leu Thr Ala Glu Arg Phe Val Ala Asp Pro Phe Val Pro Ala
   385                 390                 395                 400
   Glu Arg Met Tyr Arg Thr Gly Asp Leu Ala Tyr Trp Met Asp Gln Gly
                   405                 410                 415
   Ala Leu Ala Phe Ala Gly Arg Ala Asp Asp Gln Val Lys Ile Arg Gly
               420                 425                 430
   Tyr Arg Val Glu Pro Gly Glu Ile Glu Val Val Leu Ala Gly Leu Pro
               435                 440                 445
   Gly Val Gly Gln Ala Val Val Ser Ala Arg Asp Glu His Leu Ile Gly
       450                 455                 460
   Tyr Val Val Ala Glu Ala Gly Gln Asp Val Asp Pro Val Arg Leu Arg
   465                 470                 475                 480
   Gly Gln Leu Ala Glu Thr Leu Pro Glu Phe Met Val Pro Ala Ala Val
                   485                 490                 495
   Leu Val Leu Asp Glu Leu Pro Leu Thr Val Asn Gly Lys Val Asp Arg
               500                 505                 510
   Gln Ala Leu Pro Glu Pro Asp Phe Ala Ser Lys Ala Val Gly Arg Glu
           515                 520                 525
   Pro Ala Thr Glu Ala Glu Arg Ile Leu Cys
       530                 535
<210> 28
   <211> 1661
   <212> DNA
   <213> Artificial sequence
<220>
   <221> source
   <222> 1..1661
   <223> /organism="Artificial sequence" /note="Synthetic DNA" /mol_type="unassigned DNA"
<400> 28
   catatgctca ccgtcgcagg cgtcgaagtt actaccgccg cagagagagc attggtggcg      60
   ggtgagtggg gtgcgagcac gagcgcaccg ccgtccctgc cggcattgga tttgttcggt     120
   catcaagtgg cgcaccgtcg tgacgaaccg gcggttgtgg acggtgatcg taccgttagc     180
   tacggtgagc tggccgaacg cgcggagcgt ctggccggct acctgaacgg ccgtggcgtt     240
   cgtcgtggtg accgtgttgc tgttgtgctg gaccgtagcc cggacctgat tgcaaccctg     300
   ctggctgttt ggaaggcagg tgcggcctat gtcccggttg acccggctta ccctgtggaa     360
   cgtcgtaagt ttatgctggc tgactctggc cctgccgcgg tggtgtgcgc tgaggcatac     420
   cgcgcagcgg tgccggatac gtgtccggaa ccgatcgtgc tggatgatcc gcgcacccgc     480
   caggctgtgg cggagagccc gcgtttgagc gcaggcacct cggccgatga cctggcgtac     540
   gtgatgtaca ccagcggtag caccggcacg ccgaaaggtg tagcagtgtc tcatggcaac     600
   gtcgcggctc tggcaggtga gcctggctgg cgcgttggcc ctggcgacgc ggtcctgctg     660
   catgcgagcc acgcctttga tattagcctg ttcgagatgt gggtcccgct gctgagcggc     720
   gcacgtgttg tcctggcggg cccgggtgca gtcgatggtg cggcgctggc ggcgtatgtc     780
   gcgggtggtg tgaccgccgc acacctgacc gcgggtgctt tccgtgtgct ggcggacgag     840
   tcgccagagg cagtagcggg cctgcgtgaa gtcctgaccg gcggtgatgc ggtgccgctg     900
   gcagcggttg aacgtgtgcg tggccgtgtc cgcaatgtgc gtgttcgtca cctgtatggc     960
   ccgacggaag ctacgctgtg cgcgacgtgg tggttgctgg aaccgggtga tgagactggc    1020
   agcgtcctgc cgatcggtcg tccgctggcg ggtcgtcgtg tccatgttct ggatgcattc    1080
   ctgcgtccgg tcccaccagg tgtcgccggt gaactgtatg ttgcgggtgc aggcgttgcg    1140
   caaggttaca gcagccgtcc ggcgctgact gccgagcgtt tcgttgctga cccgtctggt    1200
   agcggtgccc gcatgtatcg cacgggtgac ctggcatact ggaccgagca gggtgcgctg    1260
   gcctttgcag gtcgtgctga cgatcaagtc aaaattcgcg gttatcgcgt tgaaccgggc    1320
   gaaattgaag tggtgctggc aggtttgccg ggtgtgggtc aagcggtcgt gacgccgcgt    1380
   ggtgaacatc tgatcggtta cgttgtggcc gaagcgggtc acgatgcgga ccctgttcgc    1440
   ctgcgcgaac agctggcggg caccctgccg gagtttatgg tcccggcagc cgtgctggtg    1500
   ttggatgagc tgccgctgac cgttaatggt aaagttgacc gtcgcgcgct gccggagccg    1560
   gatttcgcgg ccaagtccgc cggtcgcgag ccggtcacgg aggcggagcg cgttctgtgt    1620
   ggcagccgca gccaccacca tcatcaccac taacctgcag g                        1661
<210> 29
   <211> 1661
   <212> DNA
   <213> Artificial sequence
<220>
   <221> source
   <222> 1..1661
   <223> /organism="Artificial sequence" /note="Synthetic DNA" /mol_type="unassigned DNA"
<400> 29
   catatgctga gagttgccga cgtcgacgtc acgagcgctg ccgagagaga gctggtcgtc      60
   aacgaatgga gcgcagcgag ccatgcagcc ccgtcccgtc tggcaccaga cctgtttggc     120
   cgtcaagttg aacgccgtcg tgacgaagtt gccgttgttg atggcgatcg tgcgatgagc     180
   tatggcgagc tggccgaacg cgctgaaaaa ctggccggct atctgagcgg tcgcggtgtt     240
   cgccgtggtg accgtgtggc ggtggttatg gaccgcagcc cggacctgat cgctacgctg     300
   ctggcggtgt ggaaggctgg tgcggcatac gtcccggttg acccggcata cccggttgag     360
   cgcgttaagt tcatgctggc ggatgcggag ccagctgcgg tggtctgcgc ggaagcgtat     420
   cgcgacgcgg cgttggatgg tggtctggac ccgattgttt tggatgatcc gcgtacccgc     480
   caagcagttg cggagtgcac ccgtctgagc gtgggtgcga ctgcggatga cctggcttac     540
   gtgatgtata ccagcggcag cactggcacg ccgaagggtg tcgccgttag ccacggcaat     600
   gtcgccgcgt tggtgggtga gccgggctgg gcgggttccc cggacgacgc agttttgatg     660
   cacgcatccc atgcattcga catcagcctg tttgagatgt gggttccgct gttgagcggt     720
   gcacgtgttg ttctggcggg tagcggtgcc gtcgatggcg aggcactggc aggttacgta     780
   gccggtggtg tcacggccgc acacctgacg gcaggcacct ttcgtgtggt agcggaagag     840
   tctccagaaa gcatcgccgg tctgcgtgag gtgctgacgg gtggcgacgc ggtcccgcca     900
   gcggcggtgg agcgcgtccg tcgcacctgt ccgggcgttc gcgtgcgtca cctgtacggt     960
   cctaccgagg cgacgctgtg cgcgacctgg tggttgctgg agccgggtga cgaaaccggc    1020
   tccgtgctgc cgattggccg tccgctgagc ggccgtcgcg tctacgttct ggacgccttt    1080
   ctgcgtccgg tgccaccggg tgttgccggt gaactgtacg tggccggtgc cggcgtagcg    1140
   cagggctatc tgggccgcag cgcgttgacc gcagaacgtt ttgtcgcgga cccgttcgtg    1200
   cctgctgaac gtatgtatcg taccggcgat ctggcgtatt ggatggatca gggtgcactg    1260
   gcgttcgcag gtcgtgctga tgatcaggtg aaaattcgcg gttaccgcgt ggaaccgggt    1320
   gagattgagg tcgtcctggc gggtttgccg ggtgtgggcc aggcggttgt gagcgcccgt    1380
   gacgagcatt tgatcggtta cgtcgtggcg gaagctggtc aggatgttga cccagtccgt    1440
   ctgcgtggtc aactggcgga gactctgccg gagttcatgg ttccggcagc ggtgctggtc    1500
   ctggatgaac tgccgctgac cgtgaacggt aaagtggatc gtcaagcact gccggagccg    1560
   gatttcgcat ccaaagcggt cggccgtgag ccggcgaccg aagcagagcg tatcctgtgt    1620
   ggcagccgtt cgcatcatca ccaccaccac taacctgcag g                        1661
<210> 30
   <211> 73
   <212> PRT
   <213> Streptomyces lavendulae
<400> 30
   Met Thr Asn Pro Phe Asp Asn Glu Asn Gly Thr Phe Leu Val Leu Val
   1               5                   10                  15
   Asn Asp Glu Gly Gln His Ser Leu Trp Pro Val Phe Ala Glu Ile Pro
               20                  25                  30
   Gln Gly Trp Thr Thr Ala Phe Gly Glu Ala Ser Arg Ala Glu Cys Leu
           35                  40                  45
   Glu Phe Val Glu Gln Asn Trp Thr Asp Met Arg Pro Lys Ser Leu Val
       50                  55                  60
   Ala Arg Met Glu Gly Thr Ala Thr Ala
   65                  70
<210> 31
   <211> 69
   <212> PRT
   <213> Amycolatopsis balhimycina
<400> 31
   Met Ser Asn Pro Phe Asp Asn Glu Asp Gly Ser Phe Phe Val Leu Val
   1               5                   10                  15
   Asn Asp Glu Gly Gln His Ser Leu Trp Pro Thr Phe Ala Glu Val Pro
               20                  25                  30
   Ala Gly Trp Thr Arg Val His Gly Glu Ala Gly Arg Gln Glu Cys Leu
           35                  40                  45
   Ala Tyr Val Glu Glu Asn Trp Thr Asp Leu Arg Pro Lys Ser Leu Ile
       50                  55                  60
   Arg Glu Ala Ser Ala
   65
<210> 32
   <211> 69
   <212> PRT
   <213> uncultured soil bacterium
<400> 32
   Met Thr Asn Pro Phe Asp Asn Glu Asp Gly Ser Phe Phe Val Leu Val
   1               5                   10                  15
   Asn Asp Glu Gly Gln His Ser Leu Trp Pro Thr Phe Ala Glu Val Pro
               20                  25                  30
   Ala Gly Trp Val Cys Val Tyr Gly Glu Ala Thr Arg Gln Glu Cys Leu
           35                  40                  45
   Thr Phe Val Glu Glu Asn Trp Thr Asp Leu Arg Pro Lys Ser Leu Ile
       50                  55                  60
   Gln Glu Val Gly Gly
   65
<210> 33
   <211> 275
   <212> DNA
   <213> Artificial Sequence
<220>
   <221> source
   <222> 1..275
   <223> /organism="Artificial Sequence" /note="Synthetic DNA" /mol_type="unassigned DNA"
<400> 33
   ggatccagga ggacagctat gaccaaccca tttgacaacg aaaacggaac attcttagta     60
   ttagtaaacg acgaaggtca gcacagcctg tggccggtct ttgcagagat cccgcaaggt    120
   tggacgaccg cgttcggcga ggcgtcccgc gctgagtgcc tggagttcgt tgagcagaat    180
   tggaccgata tgcgtccgaa aagcctggtg gcgcgtatgg aaggtaccgc cacggcaccg    240
   ggcggccatc atcatcatca tcattgacct gcagg                               275
<210> 34
   <211> 263
   <212> DNA
   <213> Artificial sequence
<220>
   <221> source
   <222> 1..263
   <223> /organism="Artificial sequence" /note="Synthetic DNA" /mol_type="unassigned DNA"
<400> 34
   ggatccagga ggacagctat gagtaaccca tttgataatg aggacggtag tttctttgtg     60
   ttagtgaatg atgaaggtca gcacagcctg tggccgacct tcgctgaggt tccggcaggt    120
   tggacgcgtg tccatggcga ggcaggccgt caagagtgcc tggcgtacgt tgaagagaac    180
   tggaccgacc tgcgcccgaa aagcctgatc cgtgaagcca gcgcgccggg cggccatcat    240
   catcatcatc attgacctgc agg                                            263
<210> 35
   <211> 263
   <212> DNA
   <213> Artificial Sequence
<220>
   <221> source
   <222> 1..263
   <223> /organism="Artificial Sequence" /note="Synthetic DNA" /mol_type="unassigned DNA"
<400> 35
   ggatccagga ggacagctat gacgaaccca tttgataatg aggacggtag tttctttgta     60
   cttgtgaacg atgaaggtca gcacagcctg tggccgacct tcgcagaggt tccggctggc    120
   tgggtgtgcg tctacggtga agcgacccgt caggagtgtc tgacgttcgt tgaagagaat    180
   tggaccgacc tgcgcccgaa aagcctgatc caagaggtcg gcggtccggg cggccatcat    240
   catcatcatc attgacctgc agg                                            263

## Description

### Field of the invention

The present invention relates to the preparation of β-lactam antibiotics comprising contacting 4-hydroxyphenylglycine or phenylglycine, cysteine and valine with a non-ribosomal peptide synthetase and subsequent cyclization using an isopenicillin N synthase in the presence of an MbtH-like protein and to a host cell equipped to perform such preparation.

### Background of the invention

MbtH-like proteins are small proteins resembling MbtH from *Mycobacterium tuberculosis.* The function of MbtH-like proteins is, to a large extent, still unknown although recent studies indicate a role in the biosynthesis of peptides, in particular in the stimulation of adenylation reactions. Heemstra et al. (J. Amer. Chem. Soc. (2009) 131, 15317-15329) have reported adenylation of N(5)-((*R*)-3-hydroxybutyryl)-N(5)-hydroxy-D-ornithine using the adenylation domain VbsS whereby involvement of the MbtH-like protein VbsG was shown. Likewise, Felnagle et al. (Biochemistry (2010) 49, 8815-8817) have reported the adenylation of L-serine, β-lysine and L-2,3-aminopropionic acid using the adenylation domains EntF, CmnO/VioO and CmnA respectively. For L-serine adenylation the MbtH-like protein YbdZ was shown to be involved, for β-lysine these were CmnN or VioN whereas CmnN was also found to be involved in adenylation of L-2,3-aminopropionic acid. In addition MbtH-like proteins KtzJ, PacJ and GIbE were shown by Zhang et al. (Biochemistry (2010) 49, 9946-9947) to be involved in the adenylation of m-tyrosine using the adenylation domain PacL and finally it was demonstrated by Boll et al. (J. Biol. Chem. (2011) 286, 36281-36290) that MbtH-like proteins CloY, SimY and Orf1van are involved in adenylation of L-tyrosine by adenylation domains CloH, SimH or Pcza361.18.

The genes encoding MbtH-like proteins, *mbtH*-like genes, are often found in non-ribosomal peptide synthetase (NRPS) gene clusters of prokaryotic microorganisms. Many *mbtH*-like genes are deposited in GenBank. In order to identify MbtH-like proteins a BLASTP study shows homologues encoded by members of Actinobacteria, Firmacutes and Proteobacteria, however not by Archaea (R.H. Baltz, J. Ind. Microbiol. Biotechnol. (2011) 38, 1747-1760). There are no reports of *mbtH*-like genes in eukaryotic organisms.

Of the secondary metabolites produced by microorganisms, many are of significant value. An important class in this respect is that of the β-lactam antibiotics, notably the penicillins and cephalosporins. The first step in the biosynthesis of the penicillin antibiotics is the condensation of the L-isomers of three amino acids, L-α-amino adipic acid (A), L-cysteine (C) and L-valine (V) into a tripeptide, δ-(L-α-aminoadipyl)-L-cysteinyl-D-valine (ACV). This step is catalyzed by δ-(L-α-aminoadipyl)-L-cysteinyl-D-valine synthetase (ACVS). In the second step, ACV is oxidatively cyclized by the action of isopenicillin N synthase (IPNS). The product of this reaction is isopenicillin N from which the penicillins G or V are formed by exchange of the hydrophilic α-aminoadipyl side chain by a hydrophobic side chain. The side chains commonly used in industrial processes are phenylacetic acid, yielding penicillin G, or phenoxyacetic acid, yielding penicillin V. The exchange reaction is catalyzed by the enzyme acyltransferase. Due to the substrate specificity of the enzyme acyltransferase, it is hardly possible to exchange the α-aminoadipyl side chain for any other side chain of interest, although it was shown that adipic acid and certain thio-derivatives of adipic acid could be exchanged (WO 95/04148 and WO 95/04149). In particular, the side chains of industrially important penicillins and cephalosporins cannot be directly exchanged via acyltransferase. Consequently, most of the β-lactam antibiotics presently used are prepared by semi synthetic methods. These semi synthetic β-lactam antibiotics are obtained by modifying an N-substituted β-lactam product by one or more chemical and/or enzymatic reactions. These semi synthetic methods have the disadvantage that they include many steps, are not environmentally friendly and are costly. It would therefore be highly desirable to avail of a completely fermentative route to β-lactam antibiotics, for instance to amoxicillin, ampicillin, epicillin, cefadroxil, cephalexin and cephradine.

Various options can be thought of for such a completely fermentative route to semi synthetic penicillins and cephalosporins. In WO 2008/040731 it is suggested to modify the first two steps in the penicillin biosynthetic route such that amoxicillin is directly synthesized and secreted. For instance, for amoxicillin, a tripeptide comprising the amoxicillin side chain, *i.e.* D-4-hydroxyphenylglycyl-L-cysteinyl-D-valine, is constructed instead of ACV which is subsequently cyclized with a modified IPNS.

ACVS is an NRPS that catalyses the formation of the tripeptide LLD-ACV. In this tripeptide, a peptide bond is formed between the δ-carboxylic group of L-α-aminoadipic acid and the amino group of L-cysteine, and additionally the conformation of valine is changed from L to D. WO 2008/040731 discloses a modified ACVS capable of catalyzing the formation of L-4-hydroxyphenylglycyl-L-cysteinyl-D-valine and L-phenylglycyl-L-cysteinyl-D-valine (precursor for ampicillin) and capable of modifying the L stereochemical configuration of the first amino acid into a D configuration. WO 2008/040731 also discloses that native and engineered IPNS is capable of acting on D-4-hydroxyphenylglycyl-L-cysteinyl-D-valine and D-phenylglycyl-L-cysteinyl-D-valine.

Preferably the above approach is carried out in an organism capable of production under industrial conditions such as eukaryotes like Aspergillus and Penicillium. A problem associated with this approach is that yields are still low and require significant improvement.

### Detailed description of the invention

In the context of the present invention, the term "adenylation domain" refers to a protein sequence capable of recognition and activation of a specific amino acid. Preferred adenylation domains are derived from non-ribosomal peptide synthetases capable of incorporating the respective amino acids.

The term "N-α-amino-4-hydroxyphenylacetyl β-lactam antibiotic" refers to β-lactam antibiotics having a 4-hydroxyphenylglycine side chain such as amoxicillin, cefadroxil, cefatrizine, cefoperazone, cefpiramide, cefprozil, intermediates thereto and the like, preferably amoxicillin.

The term "N-α-aminophenylacetyl β-lactam antibiotic" refers to β-lactam antibiotics having a phenylglycine side chain such as ampicillin, cefaclor, cephalexin, cephaloglycine, intermediates thereto and the like, preferably ampicillin.

The term "module" defines a catalytic unit that enables incorporation of one peptide building block, usually an amino acid, in the product, usually a peptide, and may include domains for modifications like epimerization and methylation.

The term "heterologous" used in combination with modules refers to modules wherein domains, such as adenylation or condensation domains, are from different modules. These different modules may be from the same enzyme or may be from different enzymes.

The term "specific for" indicates that a module referred to as being specific for enables incorporation of the indicated amino acid.

In a first aspect of the invention there is disclosed a method for the adelylation of an 4-hydroxyphenyllycine or henylglycine comprising 4-hydroxyphenylglycine or phenylglycine, with a non-ribosomal peptide synthetase (NRPS) and ATP, characterized in that an MbtH-like protein is present wherein said MbtH-like protein has SEQ ID NO: 18, SEQ ID NO: 19, SEQ ID NO: 20, SEQ ID NO: 30, SEQ ID NO: 31 or SEQ ID NO: 32 or a sequence that is at least 50% homologous to SEQ ID NO: 18, SEQ ID NO: 19 or SEQ ID NO: 20, SEQ ID NO: 30, SEQ ID NO: 31 or SEQ ID NO: 32 or has the amino acid code NXEXQXSXWP-X₅-PXGW-X₁₃-L-X₇-WTDXRP. WO 2008/040731 A2 discloses a method for the preparation of an N-α-amino-4-hydroxyphenylacetyl or an N-α-aminophenylacetyl β-lactam antibiotic comprising the steps of (a) contacting in a eukaryotic microorganism the amino acids 4-hydroxyphenylglycine or phenylglycine, cysteine and valine with a non-ribosomal peptide synthetase to give a tripeptide 4-hydroxyphenylglycyl-cysteinyl-valine or a tripeptide phenylglycyl-cysteinyl-valine, respectively; (b) contacting the tripeptide obtained in step (a) with an isopenicillin N synthase. As part of this overall process, WO 2008/040731 A2 inherently also discloses a method for the adenylation of 4-hydroxyphenylglycine or phenylglycine comprising contacting 4-hydroxyphenylglycine or phenylglycine with a non-ribosomal peptide synthetase and ATP. The subject matter of present claim 1 differs from WO 2008/040731 A2 in that it is carried out in the presence of an MbtH-like protein having defined sequences. The objective technical problem to be solved by the present invention is the provision of a process for the adenylation of 4-hydroxyphenylglycine or phenylglycine. The solution to the problem is the provision of MbtH-like proteins in the said process. Addition of MbtH-like proteins to improve adenylation *in vitro* and *in vivo* in their original prokaryotic hosts has been implied in R.H. Baltz (J. Ind. Microbiol. Biotechnol. (2011) 38, 1747-1760), Felnagle et al. (Biochemistry (2010) 49, 8815-8817), Wenjum Zhang et al. (Biochemistry (2010) 49, 9946-9947) and Boll et al. (J. Biol. Chem. (2011) 286, 36281-36290), however these documents do not indicate that such an approach may be successful in eukaryotes nor is there an indication of the use of MbtH-like proteins in β-lactam antibiotics. In general, involvement of MbtH-like proteins in incorporation of hydroxyphenylglycine or phenylglycine has hitherto not been reported. In contrast, Stegman et al. (FEMS Microbial Letter (2006) 262, 85-92) discloses the opposite, namely that the small MbtH-like protein encoded by an internal gene of the balhimycin biosynthetic gene cluster is not required for glycopeptide production by *Amycolatopsis balhimycina,* a glycopeptide comprising hydroxyphenylglycine.

Hence, the prior art does not provide any pointers towards the use of MbtH-like proteins in the preparation of an N-α-amino-4-hydroxyphenylacetyl or an N-α-aminophenylacetyl β-lactam antibiotic. Surprisingly it was found that the incorporation of L-hydroxyphenylglycine or L-phenylglycine by the adenylation domains of the present invention is possible only in the presence of an MbtH-like protein.

In a first embodiment, preferred MbtH-like proteins are the ones described in R.H. Baltz (J. Ind. Microbiol. Biotechnol. (2011) 38, 1747-1760). More preferred MbtH-like proteins are the ones comprising invariant amino acids N17, E19, Q21, S23, W25, P26, P32, G34, W35, L48, W55, T56, D57, R59 and P60, also suitably referred to with the amino acid code NXEXQXSXWP-X₅-PXGW-X₁₃-L-X₇-WTDXRP. In the above annotation the letters D, E, G, L, N, P, Q, R, S, T, W and X refer to the commonly known single letter codes for amino acids (whereby X denotes one unspecified amino acid, X₅ denotes 5 unspecified amino acids, X₇ denotes 7 unspecified amino acids and X₁₃ denotes 13 unspecified amino acids). Preferably, the MbtH-like proteins of the present invention are those that are present in the biosynthesis clusters of which module M1 (see below) is chosen. Most preferred are Tcp13 (SEQ ID NO: 18) or Tcp17 (SEQ ID NO: 19) obtained from the teicoplanin biosynthesis cluster from *Actinoplanes teichomyceticus* (Sosio et. al., Microbiology (2004) 150, 95-102), or the MbtH-like homologue identified in the Veg biosynthesis cluster obtainable from an uncultured soil bacterium (Banik J.J. and Brady S.F., Proc. Natl. Acad. Sci. USA (2008) 105, 17273-17277) encoded by nt 33826-34035 of GenBank: EU874252 (SEQ ID NO: 20) or the MbtH-like homologue identified in the Teg biosynthesis cluster obtainable from an uncultured soil bacterium (Banik J.J. and Brady S.F., Proc. Natl. Acad. Sci. USA (2008) 105, 17273-17277) encoded by nt 33949-33158 of GenBank: EU874253 (SEQ ID NO: 32) or the MbtH-like homologue (SEQ ID NO: 31) identified in the balhimycin biosynthesis cluster from *Actinoplanes balhimycina* (Recktenwald et al., Microbiology (2002) 148, 1105-1118, Stegman et al., FEMS Microbial Lett. (2006) 262, 85-92) or the MbtH-like homologue (SEQ ID NO: 30) identified in the complestatine biosynthesis cluster from *Streptomyces lavendulae* (Chiu et al., Proc. Natl. Acad. Sci. USA (2001) 98, 8548-8553) or MbtH-like proteins having an amino sequence with a percentage identity of at least 30%, more preferably at least 40%, even more preferably at least 50%, most preferably at least 60% to said sequences. Such polypeptide modules with a percentage identity of at least 30% are also called homologous sequences or homologues.

The adenylation domain of a module determines specificity for a particular amino acid as it is responsible for recognition and activation of a dedicated amino acid and its loading of the correct amino acid onto its downstream adjacent partner thiolation domain. The adenylation reaction catalyzed by the adenylation domain is the following:

Amino acid + ATP ⇔ aminoacyl-AMP + PPi.

ATP, Mg²⁺, and amino acid are sequentially bound reversibly to the adenylation domain. Subsequently reversible breakdown of ATP by the adenylation domain into AMP is mediated by the amino acid. In this last step PPi is released. Several suitable methods for the determination of adenylation specificity are known in the art.

The classical radioactive ATP-[³²P] pyrophosphate (PPi) exchange assay (Santi et al. (Meth. Enzymol. (1974) 29, 620-627) is a common method for adenylation domain specificity determination. This method exploits the reverse reaction of AMP to ATP to quantify the interaction between the adenylation domain and the respective substrate. It uses the formation of isotopically labeled ATP, which is formed when [³²P]PPi is incorporated into AMP. The increase in labeled ATP is measured to detect the adenylation reaction (for example Recktenwald et al. (2002) Microbiology 148, 1105-1118). For the purpose of the present invention, pyrophosphate formation is analyzed using a more recently developed assay that measures the release of PPi with a method that does not require radioactive phosphates. These assays use inorganic pyrophosphatases to convert PPi produced during aminoacyl-AMP formation to orthophosphate (Pi). To measure Pi concentrations some of these assays use molybdate/malachite green reagent for colorimetric detection (McQuade *et al.* 2008) or, as used in the context of the present invention, a shift in absorbance maximum by conversion of 7-methyl-6-thioguanosine (MESG) by purine nucleoside phosphorylase (Ehmann D. E. et al. (Proc. Natl. Acad. Sci. (2000) 97, 2509-2514) or Daniel & Aldrich (Anal. Biochem. (2010) 404, 56-63)).

In order to perform these assays the corresponding enzymes preferably are present as purified proteins. Several methods are available to the skilled person in order to obtain these purified proteins. These include the heterologous over expression of the whole module comprising the adenylation domain or its single adenylation domain in a suitable host organisms like *Escherichia coli* or *Streptomyces lividans* as for example disclosed by Recktenwald et al. (Microbiology (2002) 148, 1105-1118). Preferably, these domains or modules are equipped with a tag to be used for purification by affinity chromatography. As known to the skilled person in the art these tags are useful for the characterization of the enzymes but not needed for their performance in the suitable host.

In a second embodiment, the NRPS constructs of the present invention comprise three modules, a first module M1 specific for 4-hydroxyphenylglycine and/or phenylglycine, a second module M2 specific for cysteine and a third module M3 specific for valine. The first module M1 enables incorporation of a first amino acid L-4-hydroxyphenylglycine or L-phenylglycine and, preferably, its conversion to the corresponding D-amino acid. The second module M2 enables incorporation of the amino acid L-cysteine while being coupled to the amino acid 4-hydroxyphenylglycine or phenylglycine. In particular, when the amino acid 4-hydroxyphenylglycine or phenylglycine is in its D-form, the M2 module specific for cysteine comprises a condensation domain that is D-specific for the donor and L-specific for the acceptor (^{D}C_{L}) that is fused to an adenylation domain that is heterologous thereto. The third module M3 enables incorporation of the amino acid L-valine and its conversion to the corresponding D-amino acid. In this way, the NRPS catalyzes the formation of a DLD-tripeptide D-4-hydroxyphenylglycyl-L-cysteinyl-D-valine or D-phenylglycyl-L-cysteinyl-D-valine from their L-amino acid precursors.

Each NRPS module is composed of so-called "domains", each domain being responsible for a specific reaction step in the incorporation of one peptide building block. Each module at least contains an adenylation domain, responsible for recognition and activation of an amino acid and a thiolation domain, responsible for transport of intermediates to the catalytic centers. The second and further modules in addition contain a condensation domain, responsible for formation of the peptide bond and the last module further contains a termination domain, responsible for release of the peptide. Optionally, a module may contain domains such as an epimerization domain, responsible for conversion of the L-form of the incorporated amino acid to the D-form. See Sieber et al. (Chem. Rev. (2005) 105, 715-738) for a review of the modular structure of NRPS.

In a third embodiment, a suitable source for the M1 module of the hybrid peptide synthetase of the present disclosure is an NRPS catalyzing formation of a peptide comprising the amino acid 4-hydroxyphenylglycine or phenylglycine to be incorporated as first amino acid in the peptide. Thus, a suitable M1 module is selected taking into account the nature of the amino acid to be incorporated as first amino acid of the tripeptide. In particular, the adenylation domain of a module determines selectivity for a particular amino acid. Thus, an M1 module may be selected based on the specificity of an adenylation domain for the amino acid to be incorporated. Such a selection may occur according to the specificity determining signature motif of adenylation domains as defined by Stachelhaus et al. (Chem. & Biol. (1999) 6, 493-505) and by Rausch et al. (Nucleic Acids Res. (2005), 33, 5799-5808). The M1 module does not need to contain a condensation domain or a termination domain as it is the first module of the NRPS. Thus, if present in the source module, condensation and/or termination domains may suitably be removed to obtain a first module M1 without said domains. In addition to an adenylation and a thiolation domain, the module M1 NRPS should contain an epimerization domain if an L-amino acid needs to be converted to a D-amino acid. Thus, if not present in the source module, an epimerization domain is fused to the thiolation domain of the source module to obtain a first module M1 containing adenylation, epimerization and termination domains.

Preferably, a first module M1 with 4-hydroxyphenylglycine specificity is obtainable from 4-hydroxyphenylglycine specific modules from synthetases involved in the formation of the glycopeptide antibiotic vancomycin or of the vancomycin-class compounds chloroeremomycin or balhimycin, a vancomycin synthetase, chloroeremomycin synthetase or balhimycin synthetase. Preferred modules are the fourth and fifth module of a vancomycin synthetase, chloroeremomycin synthetase, balhimycin synthetase or Veg synthetase, (and the first and the third module Veg synthetase). Preferred sources are chloroeremomycin synthetase obtainable from *Amycolatopsis orientalis* (Trauger et al., Proc. Nat. Acad. Sci. USA (2000) 97, 3112-3117), balhimycin synthetase obtainable from *Amycolatopsis balhimycina* (formerly *Amycolatopsis mediterranei*) Blp-Cluster (Recktenwald et al., Microbiology (2002) 148, 1105-1118) and Veg synthetase obtainable from an uncultured soil bacterium Veg-cluster (Banik J.J. and Brady S.F., Proc. Natl. Acad. Sci. USA (2008) 105, 17273-17277). Alternatively, 4-hydroxyphenylglycine specific modules may be obtained from synthetases involved in the formation of the lipoglycopeptide antibiotic teicoplanin or teicoplanin-class antibiotics as A47934, A40926 or Teg, a teicoplanin synthetase, A47934 synthetase, A40926 synthetase or Teg synthetase. Preferred modules are the first, fourth and fifth module of a teicoplanin synthetase, A47934 synthetase, A40926 synthetase or Teg synthetase. Preferably these modules are obtained from teicoplanin synthetase from *Actinoplanes teichomyceticus* Tcp-cluster (Sosio et. al., Microbiology (2004) 150, 95-102), A47934 synthetase obtainable from *Streptomyces toyocaensis* NRRL15009 Sta-Cluster, A40926 synthetase obtainable from *Nanomurea sp. ATCC39727* Dbv-Cluster (Sosio et. al., Chem. Biol. (2003) 10, 541-549) or a Teg synthetase obtainable from an uncultured soil bacterium Teg-cluster (Banik J.J. and Brady S.F., Proc. Natl. Acad. Sci. USA (2008) 105, 17273-17277). Alternatively, 4-hydroxyphenylglycine specific modules may be obtained from a complestatin synthetase, in particular the seventh module of a complestatin synthetase, preferably a complestatin synthetase obtainable from *Streptomyces lavendulae* (Chiu et al., Proc. Nat. Acad. Sci. USA (2001) 98, 8548-8553); Alternatively, a first module M1 with 4-hydroxyphenylglycine specificity is obtained from a CDA (Calcium-Dependent Antibiotic) synthetase and is in particular the sixth module of a CDA synthetase whereby the numbering of CDA synthetase modules as published by Hojati et al. (Chem. & Biol. (2002) 9, 1175-1187) is used. Preferably, the CDA synthetase is obtained from *Streptomyces coelicolor.*

Alternatively, for the preparation of an N-α-aminophenylacetyl β-lactam antibiotic, a first module M1 with phenylglycine specificity may be obtained from a pristinamycin synthetase, in particular the C-terminal module of the SnbD protein of pristinamycin synthetase, as published by Thibaut et al. (J. Bact. (1997) 179, 697-704). Preferably, the pristinamycin synthetase is obtainable from *Streptomyces pristinaspiralis.* The C-terminal source module from pristinamycin synthetase contains a termination domain and does not contain an epimerization domain. To prepare a module functioning as a first module in the peptide synthetase of the invention, the termination domain suitably is removed from the C-terminal source module and an epimerization domain is fused to the thiolation domain of the thus-modified C-terminal module. An epimerization domain may be obtainable from any suitable NRPS, for instance from another module of the same NRPS enzyme or from a module of a different NRPS enzyme with similar (e.g. 4-hydroxyphenylglycine or phenylglycine) or different amino acid specificity of the adenylation domain. Preferably, the epimerization domain is obtainable from a CDA Synthetase from *Streptomyces coelicolor,* more preferably from the sixth module, as specified above. Thus, in this embodiment, the module M1 of the NRPS is a hybrid module. The epimerization domains described above may also be fused to those modules M1 with 4-hydroxyphenylglycine specificity lacking an epimerization domain as described in the first embodiment.

Unexpectedly, it is found that several modules M1 with 4-hydroxyphenylglycine specificity as described in the first embodiment are capable of activating L-phenylglycine in the presence of MbtH-like proteins and are therefore suitable for use as first module M1 in the construction of NRPS constructs designed for N-α-aminophenylacetyl β-lactam antibiotics. These modules are for example the first module of a teicoplanin synthetase, A47934 synthetase or A40926 synthetase. Preferably these first modules are obtained from teicoplanin synthetase from *Actinoplanes teichomyceticus* Tcp-cluster (Sosio et. al., Microbiology (2004) 150, 95-102), A47934 synthetase obtainable from *Streptomyces toyocaensis* NRRL15009 Sta-Cluster or A40926 synthetase obtainable from *Nanomurea* sp. ATCC39727 Dbv-Cluster (Sosio et. al., Chem. Biol. (2003) 10, 541-549). These modules are further the third module of a teicoplanin synthetase, or a Veg synthetase. Preferably, these first modules are obtained from teicoplanin synthetase from *Actinoplanes teichomyceticus* Tcp-cluster (Sosio et. al., Microbiology (2004) 150, 95-102), or Veg synthetase obtainable from an uncultured soil bacterium Veg-cluster (Banik J.J. and Brady S.F., Proc. Natl. Acad. Sci. USA (2008) 105, 17273-17277). These modules are further the fifth module of a chloroeremomycin synthetase, or balhimycin synthetase. Preferred sources for the fifth module are chloroeremomycin synthetase obtainable from *Amycolatopsis orientalis* (Trauger et al., Proc. Nat. Acad. Sci. USA (2000) 97, 3112-3117), and balhimycin synthetase obtainable from *Amycolatopsis balhimycina* (formerly *Amycolatopsis mediterranei*) Blp-Cluster (Recktenwald et al., Microbiology (2002) 148, 1105-1118).

In a fourth embodiment, the second module M2 of the peptide synthetase should enable incorporation of the amino acid cysteine as second amino acid of the tripeptide DLD-XCV, wherein X is 4-hydroxyphenylglycine or phenylglycine. Selection of this module may be based on the specificity determining signature motif of adenylation domains as published by Stachelhaus et al. (Chem. & Biol. (1999) 8, 493-505). An example for the second module M2 is the first module of the peptide synthetase
L- L-
- Watanabe et al. (Curr. Opin. Chem. Biol. (2009) 13, 189-196).

To enable coupling of the L-cysteinyl acceptor to the D-X-aminoacyl donor, the condensation domain of the M2 module is a ^{D}C_{L} domain, as outlined above and as explained in Clugston et al. (Biochemistry (2003) 42, 12095-12104). This ^{D}C_{L} domain is fused to an adenylation domain that is heterologous thereto. The hybrid M2 module comprising such a ^{D}C_{L}-adenylation domain configuration appears capable of incorporation of the amino acid cysteine. In a preferred embodiment, the ^{D}C_{L} domain of the M2 module is obtainable from the module immediately downstream of the module that is the source of the first module M1 of the peptide synthetase of the invention. For instance, the ^{D}C_{L} domain of the M2 module of the peptide synthetase is the ^{D}C_{L} domain of the seventh module of the CDA synthetase that is the source of the first module M1. In another embodiment, the ^{D}C_{L} domain of the M2 module of the peptide synthetase is the ^{D}C_{L} domain of the second module of the *Bacillus subtilis* RB14 Iturin Synthetase Protein ItuC, as defined by Tsuge et al. (J. Bacteriol. (2001) 183, 6265-6273). In a preferred embodiment of the invention, the second module M2 of the peptide synthetase is at least partly obtainable from the enzyme that is the source of the third module M3 of the peptide synthetase. In particular, the adenylation and thiolation domains of the M2 module of the peptide synthetase are obtainable from the module immediately upstream of the module that is the source of the third module of the peptide synthetase of the invention. For instance, the adenylation and thiolation domains of the M2 module of the peptide synthetase may be the adenylation and thiolation domains of the second module of an ACVS.

In a fifth embodiment, the third module M3 of the peptide synthetase enables incorporation of the amino acid valine as the third amino acid of the tripeptide, as well as its conversion to the D-form, to yield the tripeptide DLD-XCV. An example for the third module M3 is the second module of the peptide synthetase
L- L-
- Watanabe et al. (Curr. Opin. Chem. Biol. (2009) 13, 189-196) and an epimerization domain is fused to the thiolation domain. An epimerization domain may be obtainable from any suitable NRPS, for instance from another module of the same NRPS enzyme or from a module of a different NRPS enzyme with similar (e.g. L-valine) or different amino acid specificity of the adenylation domain. In a preferred embodiment of the invention, the third module of the peptide synthetase is obtainable from an ACVS and preferably is the third module of an ACVS. The ACVS as mentioned above preferably is a bacterial or fungal ACVS, more preferably a bacterial ACVS obtainable from *Nocardia lactamdurans* or a fungal ACVS obtainable from a filamentous fungus such as *Penicillium chrysogenum, Acremonium chrysogenum, and Aspergillus nidulans.*

The modules M1, M2 and M3 of the peptide synthetase may have the amino acid sequences as disclosed in WO 2008/040731. Hence, the M1 module of the peptide synthetase for instance has an amino acid sequence according to SEQ ID NO: 2 or SEQ ID NO: 4 of WO 2008/040731, or contains SEQ ID NO: 1 - SEQ ID NO: 9 of the present invention, or has an amino sequence with a percentage identity of at least 30%, more preferably at least 40%, even more preferably at least 50%, most preferably at least 60% to said sequences. Such polypeptide modules with a percentage identity of at least 30% are also called homologous sequences or homologues. Likewise, the M2 module of the peptide synthetase for instance has an amino acid sequence according to SEQ ID NO: 6 or to SEQ ID NO: 8 of WO 2008/040731 or an amino sequence with a percentage identity of at least 30%, more preferably at least 40%, even more preferably at least 50%, most preferably at least 60% to said sequences. Finally, the M3 module of the peptide synthetase for instance has an amino acid sequence according to SEQ ID NO: 10 of WO 2008/040731 or an amino sequence with a percentage identity of at least 30%, more preferably at least 40%, even more preferably at least 50%, most preferably at least 60% to said sequence.

The modules of the NRPS constructs of the present invention may be obtained as disclosed in WO 2008/040731. Typically, the adenylation domain of a module determines specificity for a particular amino acid; whereas epimerization and condensation domains may be obtained form any module of choice. Engineered NRPS enzymes may be constructed by fusion of the appropriate domains and/or modules in the appropriate order. It is also possible to exchange a module or domain of an enzyme for a suitable module or domain of another enzyme. This fusion or exchange of domains and/or modules may be done using genetic engineering techniques commonly known in the art. Fusion of two different domains or modules may typically be done in the linker regions that are present in between modules or domains. See for instance EP 1255816 and Mootz et al. (Proc. Natl. Acad. Sci. USA, (2000) 97, 5848-5853) disclosing these types of constructions. Part or all of the sequences may also be obtained by custom synthesis of the appropriate polynucleotide sequence(s).

For instance, the fusion of an adenylation-thiolation-epimerization tri-domain fragment from a 4-hydroxyphenylglycine specific NRPS module to the bi-modular cysteine-valine specific fragment of an ACVS may be done by isolation using restriction enzyme digestion of the corresponding NRPS gene at the linker positions, more specifically, between the condensation domain and the adenylation domain of the 4-hydroxyphenylglycine specific module, in case of a C-terminal module or between the condensation domain and the adenylation domain of the 4-hydroxyphenylglycine specific module and between the epimerization domain and the subsequent domain (condensation or termination domain), in case of an internal elongation module. The bi-modular cysteine-valine specific fragment of ACVS may be obtained by 1) leaving the C-terminus intact, and 2) exchanging the condensation domain of the cysteine specific module 2 for a condensation domain which has ^{D}C_{L} specificity. In analogy to isolation of the adenylation-thiolation-epimerization fragment, an adenylation-thiolation-epimerization-condensation four-domain fragment may be isolated including the condensation domain of the adjacent downstream module. The latter is fused to the bi-modular cysteine-valine specific fragment of ACVS without the upstream condensation domain.

In a sixth embodiment, the NRPS enzymes as described herein may be suitably subjected to mutagenesis techniques, e.g. to improve the catalytic properties of the enzymes. Polypeptides as described herein may be produced by synthetic means although usually they will be made recombinantly by expression of a polynucleotide sequence encoding the polypeptide in a suitable host organism. Polynucleotides encoding the NRPS constructs of the present invention, polypeptides with improved activity and vectors comprising said polynucleotides are obtained as described in WO 2008/040731.

In a second aspect of the invention there is provided a host cell transformed with or comprising a polynucleotide or vector as described in WO 2008/040731 combined with a polynucleotide according to the present invention allowing the expression of an MbtH-like protein. Suitable host cells are host cells that allow for a high expression level of a polypeptide of interest. Such host cells are usable in case the polypeptides need to be produced and further to be used, e.g. in *in vitro* reactions. A heterologous host may be chosen wherein the polypeptides of the invention are produced in a form that is substantially free from other polypeptides with a similar activity as the polypeptide of the invention. This may be achieved by choosing a host that does not normally produce such polypeptides with similar activity. Suitable host cells also are cells capable of production of β-lactam compounds, preferably host cells possessing the capacity to produce β-lactam compounds in high levels. The host may be selected based on the choice to produce a penicillin or cephalosporin compound.

In one embodiment, a suitable host cell is a cell wherein the native genes encoding the ACVS and/or IPNS enzymes are inactivated, for instance by insertional inactivation. It is also possible to delete the complete penicillin biosynthetic cluster comprising the genes encoding ACVS, IPNS and AT. In this way the production of the β-lactam compound of interest is possible without simultaneous production of the natural β-lactam. Insertional inactivation may thereby occur using a gene encoding a NRPS and/or a gene encoding an IPNS as described above. In host cells that contain multiple copies of β-lactam gene clusters, host cells wherein these clusters are spontaneously deleted may be selected. For instance, the deletion of β-lactam gene clusters is described in WO 2007/122249.

Another suitable host cell is a cell that is capable of synthesizing the precursor amino acids 4-hydroxyphenylglycine or phenylglycine. Heterologous expression of the genes of the biosynthetic pathway leading to 4-hydroxyphenylglycine or phenylglycine is disclosed in WO 2002/034921. The biosynthesis of 4-hydroxyphenylglycine or phenylglycine is achieved by withdrawing 4-hydroxyphenylpyruvate or phenylpyruvate, respectively, from the aromatic amino acid pathway, converting said components to 4-hydroxymandelic acid or mandelic acid, respectively, subsequently converting to 4-hydroxyphenylglyoxylate or phenylglyoxylate, respectively and finally converting to D-4-hydroxyphenylglycine or D-phenylglycine, respectively.

Another suitable host cell is a cell that (over) expresses a 4'-phosphopantetheine transferase. 4'-Phosphopantetheine is an essential prosthetic group of amongst others acyl-carrier proteins of fatty acid synthases and polyketide synthases, and peptidyl carrier proteins of NRPS's. The free thiol moiety of 4'-phosphopantetheine serves to covalently bind the acyl reaction intermediates as thioesters during the multistep assembly of the monomeric precursors, typically acetyl, malonyl, and aminoacyl groups. The 4'-phosphopantetheine moiety is derived from coenzyme A and post translationally transferred onto an invariant serine side chain. This Mg²⁺-dependent conversion of the apoproteins to the holoproteins is catalyzed by the 4'-phosphopantetheine transferases. It is advantageous to (over)express a 4'-phosphopantetheine transferase with a broad substrate specificity. Such a 4'-phosphopantetheine transferase is for instance encoded by the gsp gene from *Bacillus brevis* as described by Borchert et al. (J. Bacteriol. (1994) 176, 2458-2462).

A host may suitably include one or more of the modifications as mentioned above. A preferred host is an organism capable of production under industrial conditions such as eukaryotes like Penicillium, Acremonium and Aspergillus examples of which are *Penicillium chrysogenum, Acremonium chrysogenum, and Aspergillus nidulans.*

### Legend to the Figures

Figures 1 to 4 depict the adenylation activity measurements with PPi Release assay for substrates L-phenylalanine (□), D-phenylalanine (■), L-hydroxyphenylglycine (●) and D-hydroxyphenylglycine (▲) normalized for the incubation without substrate. X-axis: time (min); Y-axis: absorption (360 nm).
- Figure 1:: For control protein TycA
- Figure 2:: For StaA_M1_A
- Figure 3:: ForVeg8_M1_A
- Figure 4:: For Veg8_M1_A and Tcp13

### Examples

### General material and methods

### Molecular and genetic techniques

Standard genetic and molecular biology techniques are known in the art (*e.g*. Maniatis et al. "Molecular cloning: a laboratory manual" (1982) Cold Spring Harbor Laboratory, Cold Spring Harbor, N.Y.; Miller "Experiments in molecular genetics" (1972) Cold Spring Harbor Laboratory, Cold Spring Harbor, N.Y.; Sambrook and Russell "Molecular cloning: a laboratory manual" (3rd edition)" (2001) Cold Spring Harbor Laboratory, Cold Spring Harbor Laboratory Press; Ausubel "Current protocols in molecular biology" (1987) Green Publishing and Wiley Interscience, New York).

### Plasmids and Strains

pMAL-c5x was obtained from New England Biolabs Inc., pACYCtac has been described previously (M. Krämer "Untersuchungen zum Einfluss erhöhter Bereitstellung van Erythrose-4-Phosphat und Phosphoenolpyruvat auf den Kohlesrofffluss in den Aromatenbiosyntheseweg von Escherichia coli", Berichte des Forschungszentrums Jülich, 3824, ISSN 0944-2952 (PhD Thesis, University of Düsseldorf). *Escherichia coli* strains Top10 (Invitrogen, Carlsbad, CA, USA) or DH10b (Grant et al. (1990) Proc. Natl. Acad. Sci. USA (1990) 87, 4645-4649) were used for cloning and protein expression. *Escherichia coli* strain M15 pQE60-tycA pRep4 as described in Mootz, H. D. et al. (Proc. Natl. Acad. Sci. USA (2000) 97, 5848-53) and Mootz H.D. and Marahiel, M. A. (J Bacteriol. (1997) 179, 6843-6850) was kindly provided by Prof. M. Marahiel, Philipps University Marburg, Marburg, Germany.

### Media

2xPY medium (16 g/l BD BBL™ Phytone™ Peptone, 10 g/l Yeast Extract, 5 g/l NaCl) was used for growth of *Escherichia coli.* Antibiotics (100 µg/ml ampicillin, or 50 µg/ml ampicillin together with 20 µg/ml chloramphenicol, or 100 µg/ml ampicillin together with 25 µg/ml neomycin depending on plasmids used) were supplemented to maintain plasmids. For induction of gene expression IPTG was used at 0.03-0.5 mM final concentration.

### Identification of Plasmids

Plasmids carrying the different genes were identified by genetic, biochemical and/or phenotypic means generally known in the art, such as resistance of transformants to antibiotics, purification of plasmid DNA, restriction analysis of purified plasmid DNA or DNA sequence analysis.

### Collection of putative HPG adenylation domains from existing NRPS sequences in Uniprot/NCBI-ENV-PAT databases

**Table 1**

| Uniprot code | Encoded protein | Module number in encoded protein predicted to be specific for HPG | Module number in predicted biosynthesis cluster | SEQ ID NO: adenylation domain | Organism |
|---|---|---|---|---|---|
| Q70AZ9 | Tcp9 | M1 | M1 | 1 | *Actinoplanes teichomyceticus* |
| Q7WZ66 | Dbv25 | M1 | M1 | 2 | *Nonomuraea sp. ATCC* 39727 |
| Q8KLL3 | StaA | M1 | M1 | 3 | *Streptomyces toyocaensis* |
| 052820 | CepB (PCZA363.4) | M2 | M5 | 4 | *Amycolatopsis orientalis* |
| Q939Z0 | BpsB | M2 | M5 | 5 | *Amycolatopsis balhimycina* |
| B7T1C1 | Veg8 | M1 | M4 | 6 | *uncultured soil bacterium* |
| Q70AZ7 | Tcp11 | M1 | M4 | 7 | *Actinoplanes teichomyceticus* |
| Q8KLL5 | StaC | M2 | M5 | 8 | *Streptomyces toyocaensis* |
| Q93N88 | ComB | M1 | M3 | 9 | *Streptomyces lavendulae* |
| Q939Z0 | BpsB | M1 | M4 | 26 | *Amycolatopsis balhimycina* |
| B7T1D2 | Teg7 | M1 | M4 | 27 | *uncultured soil bacterium* |

All proteins simultaneously containing the Pfam profiles characteristic for adenylation domains (Pfam identifier AMP-binding), Phosphopanthetheinyl-binding (Pfam identifier PP-binding) and condensation domains (Pfam identifier condensation) were collected from UniRef100 and NCBI env_nr and protein databases. These proteins are putative NRPS proteins. Putative NRPS protein sequences were selected from UniRef100 and NCBI env_nr and patent protein databases. Putative HPG adenylation domains were selected from NRPS's. In addition to predictions by the program NRPSpredictor (Rausch et al. (Nucleic Acids Res. (2005), 33, 5799-5808), the so-called Stachelhaus code (10 amino acids closest to the substrate bound in the active site (Stachelhaus et al. (Chem. & Biol. (1999) 6, 493-505)) was used, to predict the preferred amino acid bound by the adenylation domain of the identified NRPS Synthetase. Of the adenylation domains predicted to prefer 4-hydroxyphenylglycine, the following selection (Table 1) was made for biochemical characterization of adenylation specificity.

### Example 1

### Synthetic design, cloning, expression, and purification of NRPS adenylation domains which are predicted as being specific for L-hydroxyphenylglycine in Escherichia coli

### Expression constructs

Synthetic constructs codon optimized for *Escherichia coli* were designed for the adenylation domains with SEQ ID NO: 2-9, SEQ ID NO: 26, and SEQ ID NO: 27 as given above resulting in nucleotide SEQ ID NO: 10-17, SEQ ID NO: 28, and SEQ ID NO: 29, and ordered at DNA2.0. All were equipped with a C-terminal 6*His-tag for subsequent affinity chromatography (in appending a nucleotide sequence encoding the amino acid sequence GSRSHHHHHH) at the C terminus of the recombinant protein and flanked by restriction enzyme cloning sites Ndel/Sbfl for subsequent cloning in the Ndel/Sbfl sites of expression vector pMAL-c5x. The cloning of the synthetic DNA fragments in this vector results in the expression of a fusion protein of the respective A-domain with maltose binding protein at the N-terminus which allows high level of soluble protein expression by *Escherichia coli.* The final plasmids for overexpression of the adenylation domains constructed by cloning the Ndel/Sfbl fragments taken from the synthetic constructs provided bt DNA2.0 into the Ndel/Sbfl sites of expression vector pMAL-c5x were named pMAL-Dbv25_M1_A, pMAL-StaA_M1_A, pMAL-CepB_M2_A, pMAL-BpsB_M2_A, pMAL-Veg8_M1_A, pMAL-Tcp11_M1_A, pMAL-StaC_M2_A, pMAL-ComB_M1_A, pMAL-BpsB_M1_A, pMAL-Teg7_M1_A. In case of the construction of plasmid pMAL-StaA_M1_A, cloning by partial digestions of the synthetic construct SEQ ID NO: 11 with Sbfl needed to be performed as the ordered fragment contained by mistake an additional Sfbl site.

### Protein expression in Escherichia coli

Starter cultures of *Escherichia coli* harbouring plasmid pMAL-Dbv25_M1_A, or pMAL-StaA_M1_A, or pMAL-CepB_M2_A, or pMAL-BpsB_M2_A, or pMAL-Veg8_M1_A, or pMAL-Tcp11_M1_A, or pMAL-StaC_M2_A, or pMAL-ComB_M1_A, or pMAL-BpsB_M1_A, or pMAL-Teg7_M1_A were grown overnight at 37°C in 3 ml 2*PY medium with 100 µg/ml ampicillin. The next day 100 ml 2*PY medium with 100 µg/ml ampicillin in 0.5 l shake flask was inoculated with the preculture to an OD₆₀₀ₙₘ of 0.015 and grown at 30°C and 280 rpm. When an OD₆₀₀ₙₘ of 0.4 - 0.6 was reached, the shake flask was cultured at 18°C and 280 rpm for one hour. Following this temperature (pre-) adaptation, 3 µl of 1 M IPTG was added and the culture was grown at 18°C and 220 rpm overnight.

### Preparation of cell free extracts and His-tag purification:

Cells from 50 ml of the cultivations described in previous paragraph were harvested by centrifugation (5000 rpm, 10 minutes, 4°C) and the pellets were re-suspended in 1 ml extraction buffer (50 mM Hepes pH 8.0, 5 mM DTT, 100 mM NaCl, 1x EDTA-free Complete protease inhibitor cocktail (Roche)). Cell lysis was obtained by sonification (9x 10 sec. on / 15 sec. off) keeping cells on ice during the procedure. To remove cell debris, the sonificated samples were centrifuged at 14.000 rpm for 15 min at 4°C and the supernatants (cell free extracts) with the soluble proteins were transferred to fresh vials and kept on ice until further use. For purification of the His-tagged proteins TALON® Metal Affinity Resin was used according to the manufacturer's protocol (Clontech Laboratories, Inc. US; Protocol No. PT1320-1, Version No. PR6Z2142, page 30; VIII B Batch/Gravity-Flow Column Purification). Equilibration and washing of the column material was done with 50 mM Hepes pH8.0. Elution was done with 50 mM Hepes pH8.0 + 150 mM imidazole. 1 ml fractions were collected and kept on ice. The purified proteins are designated as Dbv25_M1_A, StaA_M1_A, CepB_M2_A, BpsB_M2_A, Veg8_M1_A, Tcp11_M1_A, StaC_M2_A, ComB_M1_A, BpsB_M1_A, or Teg7_M1_A.

### Analyses purified proteins

By use of SDS-PAGE analysis (NuPAGE gels used according to manufacturers protocol) cell free extracts and the different elution fractions collected from the His-tag purification were analyzed for the presence of proteins and of correct size corresponding to the adenylation domains. For all adenylation domains over expressed, purification of a protein of the respective size was confirmed. The protein concentration of the different samples was determined using Coomassie Plus™ (Bradford) Assay Reagent (Thermo Scientific, PIERCE) according to the manufacturer's protocol.

### Example 2

### Expression and purification of TycA comprising adenylation domain specific for phenylalanine as internal control for adenylation activity assay

*Escherichia coli* strain M15 pQE60-tycA pRep4 (see Plasmids and Strains) was used for overexpression and purification of TycA the first one-module-bearing peptide synthetase for synthesis of tyrocidine by *Bacillus brevis.* Expression and purification of TycA was performed as described in example 1, with the following variations. Antibiotics used in the medium were 100 µg/ml ampicillin and 25 µg/ml neomycin. Induction was done when the main culture was grown at 30°C and 280 rpm to an OD₆₀₀ of 0.4 - 0.6 by addition of 50 µl of 1 M IPTG. After induction the cells were grown for additional 3 hours at 30°C and 280 rpm before they were harvested. Preparation of cell lysates and protein purification was performed as described in Example 1.

### Example 3

### Synthetic design and cloning of MbtH-like proteins Tcp11, Tcp13 from teicoplanin cluster and VMbtH from Veg-Cluster

Three different MbtH-like proteins were chosen, two from the teicoplanin biosynthetic cluster annotated as tcp13 (SEQ ID NO: 18, GenBank: AJ605139 Genomic DNA; Translation: CAE53354.1) and tcp17 (SEQ ID NO: 19, GenBank: AJ605139 Genomic DNA; Translation: CAE53358.1) and one from the Veg biosynthetic clusters. The last one was named VMbtH, as it is not annotated in public databases yet and was identified by a search for homologous MbtH-like sequences in the Veg Cluster (SEQ ID NO: 20, GenBank: EU874252, nt 33826-34035, between veg9 and veg10). Target genes encoding the selected proteins were constructed synthetically (DNA2.0) resulting in nucleotide SEQ ID NO: 21-23 and ordered at DNA2.0. The genes encoding Tcp13 and Tcp17 were chosen as their wild type sequence, while the gene encoding VMbtH was codon optimized for expression in *Escherichia coli.* Each ORF was preceded by a consensus ribosomal binding site and flanked by restriction sites BamHI and Sbfl for final cloning in expression plasmid pACYCtac. The final plasmids for overexpression of the MbtH-like proteins constructed by cloning the BamHI/Sbfl fragments taken from the synthetic constructs provided bt DNA2.0 into the BamHI/Sbfl sites of expression vector pACYCtac were named pACYCtac-Tcp13, pACYCtac-Tcp17 and pACYCtac-VMbtH.

### Example 4

### Synthetic design and cloning of MbtH-like proteins from complestatine, balhimycin and Teg-Cluster

Three additional MbtH-like proteins were chosen, one from the complestatine biosynthetic cluster annotated as hypothetical protein (SEQ ID NO: 30, GenBank: AF386507 Genomic DNA; Translation: AAK81828.1) and called CMbtH, one from the balhimycin biosynthetic cluster annotated as hypothetical protein and called BMbtH (SEQ ID NO: 31, GenBank: Y16952.3 Genomic DNA; Translation: CAC48363.1) and called BMbtH, and one from the Teg biosynthetic clusters. The last one is not annotated in public databases yet and was identified by a search for homologous MbtH-like sequences in the Teg Cluster (SEQ ID NO: 32, GenBank: EU874253, nt 32949-33158, between teg8 and teg9). It was called TMbtH. Target genes encoding the selected proteins were constructed synthetically (DNA2.0) resulting in nucleotide SEQ ID NO: 33-35 and ordered at DNA2.0 codon optimized for expression in *Escherichia coli.* All were equipped with a C-terminal 6*His-tag for possible affinity chromatography (in appending a nucleotide sequence encoding the amino acid sequence PGGHHHHHH) at the C terminus of the recombinant protein. Each ORF was preceded by a consensus ribosomal binding site and flanked by restriction sites BamHI and Sbfl for final cloning in expression plasmid pACYCtac. The final plasmids for overexpression of the MbtH-like proteins constructed by cloning the BamHI/Sbfl fragments taken from the synthetic constructs provided bt DNA2.0 into the BamHI/Sbfl sites of expression vector pACYCtac were named pACYCtac-BMbtH, pACYCtac-CMbtH and pACYCtac-TMbtH.

### Example 5

### Co-expression and co-purification of adenylation domains with MbtH like proteins

*Escherichia coli* strains harboring a pMAL plasmid for over expression of an adenylation domain as described in Example 1 and a pACYCtac plasmid for over expression of a MbtH-like protein as described in Example 3 and Example 4 were used for co-expression and co-purification of these two proteins. Expression and purification of an adenylation domain together with an MbtH-like protein was performed as described in Example 1, except that antibiotics used in the medium were 50 µg/ml ampicillin and 20 µg/ml chloramphenicol. By SDS page analysis of the elution fractions as described in Example 1, purification of two separate proteins was confirmed, one comprising the size of the respective adenylation domain, and another comprising the size of the MbtH-like protein. As the MbtH-like proteins Tcp13, Tcp17 and VMbtH are not equipped with a His-tag but nevertheless co-purified with the coexpressed adenylation domain, both proteins are tighly bound.

### Example 6

### Synthetic design, cloning, expression, and purification of an NRPS adenylation-thiolation didomain with and without MbtH-like proteins

### Expression constructs

A synthetic construct was designed for the adenylation thiolation didomain comprising the wild type nucleotide sequence encoding SEQ ID NO: 1 together with its adjacent thiolation domain present in the Tcp9 encoding protein. This construct was equipped with a C-terminal 6*His-tag for subsequent affinity chromatography (in appending a nucleotide sequence encoding the amino acid sequence GSRSHHHHHH) at the C terminus of the recombinant protein and flanked by restriction enzyme cloning sites Ndel/Sbfl for subsequent cloning in the Ndel/Sbfl sites of expression vector pMAL-c5x. Cloning of the synthetic DNA fragment in this vector results in the expression of a fusion protein of the respective AT-didomain with maltose binding protein at the N-terminus which allows high level of soluble protein expression by *Escherichia coli.* The final plasmid for overexpression of the adenylation thiolation didomain constructed by cloning the Ndel/Sfbl fragments taken from the synthetic constructs SEQ ID NO: 24 provided by DNA2.0 into the Ndel/Sbfl sites of expression vector pMAL-c5x was named pMAL-Tcp9_M1_AT.

Protein expression and purification of the separate adenylation thiolation didomain was performed as described in Example 1, the purified protein was designated as Tcp9_M1_AT. Protein co-expression and co-purification of adenylation thiolation didomain together with an MbtH-like protein was performed as described in Example 5. By SDS page analysis of the elution fractions as described in sample 1, purification of either the separate adenylation thiolation didomain or two separate proteins was confirmed, one protein comprising the size of the respective adenylation thiolation didomain, and one protein comprising the size of the MbtH-like protein. As the MbtH-like proteins Tcp13, Tcp17 and VMbtH are not foreseen with a His-tag but nevertheless purified together with the adenylation thiolation didomain, both proteins are tighly bound.

### Example 7

### Synthetic design, cloning, expression, and purification of an NRPS adenylation-thiolation-epimerization tridomain with and without MbtH-like proteins

### Expression constructs

A synthetic construct codon optimized for *Escherichia coli* was designed comprising the adenylation domain with SEQ ID NO: 6 and its adjacent thiolation domain and epimerization domain present in the Veg8 encoding protein. This construct was equipped with a C-terminal 6*His-tag for subsequent affinity chromatography (in appending a nucleotide sequence encoding the amino acid sequence GSRSHHHHHH) at the C terminus of the recombinant protein and flanked by restriction enzyme cloning sites Ndel/Sbfl for subsequent cloning in the Ndel/Sbfl sites of expression vector pMAL-c5x. Cloning of the synthetic DNA fragment in this vector results in the expression of a fusion protein of the respective ATE-tridomain with maltose binding protein at the N-terminus which allows high level of soluble protein expression by *Escherichia coli.* The final plasmid for overexpression of the adenylation thiolation didomain constructed by cloning the Ndel/Sfbl fragments taken from the synthetic constructs SEQ ID NO: 25 provided by DNA2.0 into the Ndel/Sbfl sites of expression vector pMAL-c5x was named pMAL-Veg8_M1_ATE.

Protein expression and purification of the separate adenylation thiolation epimerization tridomain was performed as described in Example 1, the purified protein was designated as Veg8_M1_ATE. Protein co-expression and co-purification of adenylation thiolation epimerization tridomain together with an MbtH-like protein was performed as described in Example 5.

By SDS page analysis of the elution fractions as described in sample 1, purification of either the separate adenylation thiolation didomain or two separate proteins was confirmed, one protein comprising the size of the respective adenylation thiolation didomain, and one protein comprising the size of the MbtH-like protein. As the MbtH-like proteins Tcp13, Tcp17 and VMbtH are not foreseen with a His-tag but nevertheless purified together with the adenylation thiolation epimerization tridomain, both proteins are tighly bound.

### Example 8

### Determination of adenylation activity for putative L-hydroxyphenylglycine adenylation domains, an adenylation thiolation didomain and an adenylation thiolation epimerization tridomain by PPi release assay

To determine the adenylation activity of the adenylation domains, the Enzchek® pyrophosphate assay kit (Life Technologies) was used as described by Ehmann D. E. et al. (Proc Nat Acad Science (2000) 97, 2509-2514) with small modifications. The reactions were performed 96 wells UV/Vis transparent plates (BD Falcon). The reaction mixture comprises 50 mM HEPES pH 8.0, 10 mM MgCl2, 5 mM ATP, 75 mM DTT, 0.03 U Inorganic Pyrophosphatase (IP), 1 U Purine Nucleoside Phosphorylase (PNP) and 0.2 mM MESG in a volume of 70 µl. Next 20 µl (around 0.5-2 µM final concentration) of purified A(T) domain, with or without co-purification of the MbtH like helper protein was added and the reaction was pre-incubated for 15 minutes at RT to reduce contaminating Pi. Following the pre-incubation, 10 µl of a 10 mM or 1 mM solution of the appropriate amino acid depending on the performed specificity determination was added to initiate the adenylation reaction and the absorbance at 360 nm was measured using a TECAN I Control spectrophotometer. Absorbance measurements were made every 5 to 10 min over a period of up to 240 min. A reaction with addition of 10 µl MilliQ water instead was used to determine and subtract the background absorbance. As substrates the following amino acids were used: D- or L-phenylalanine, D- or L-hydroxyphenylglycine, D- or L-phenylglycine, L-tryptophan, L-valine, L-cysteine, and L-leucine.

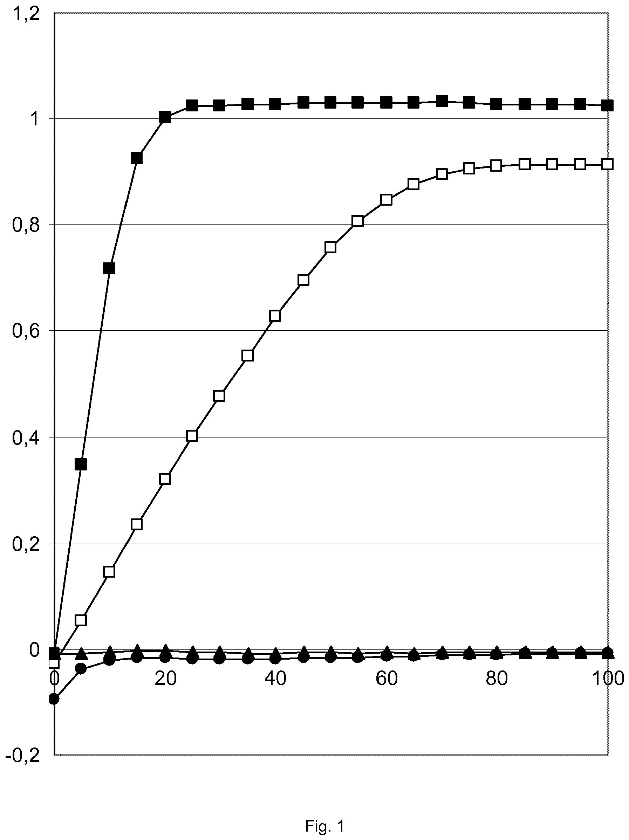

Figure 1 shows a graph of the absorption measurements of the PPi release assay with the control protein TycA. While L- and D-phenylalanine are accepted as substrate, no adenylation activity is measured for L- and D-hydroxyphenylglycine. Beside L- and D-phenylalanine, also L-tryptophan, L-valine and L-leucine (data not shown) have been shown to be similarly recognized and adenylated by TycA while no adenylation activity was measured for L-cysteine (data not shown) which is in agreement with the findings of Villiers and Hollfelder (ChemBioChem (2009) 10, 671 -682).

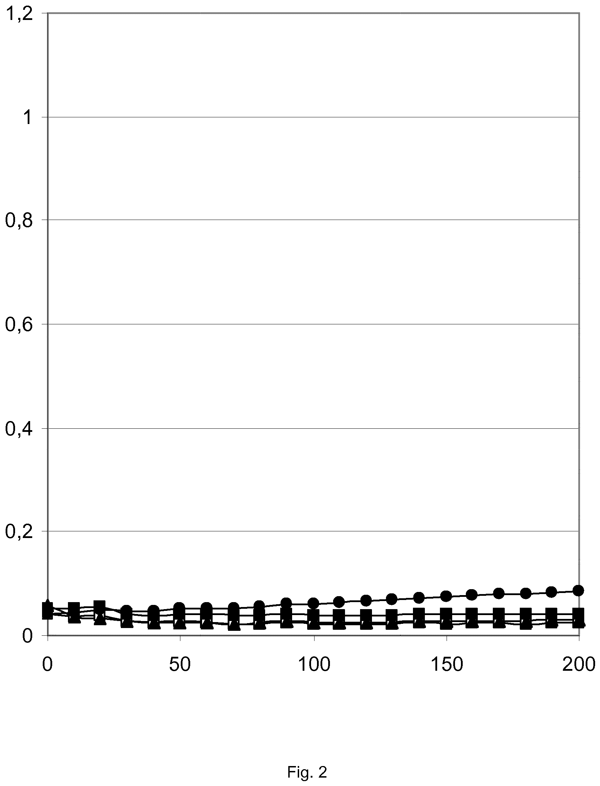

Figure 2 shows a graph for the absorption measurements of the PPi release assay with the single adenylation domain derived from StaA_M1_A. No adenylation activity is determined for the amino acids L- or D-hydroxyphenylglycine, nor L- or D-phenylalanine.

The graphs for the adenylation domains Dbv25_M1_A, CepB_M2_A, BpsB_M2_A, Tcp11_M1_A, StaC_M2_A, ComB_M1_A, BpsB_M1_A, Teg7_M1_A, or the adenylation thiolation didomain of Tcp9_M1_AT gave the same results (data not shown). No adenylation activity could be confirmed for L- or D-hydroxyphenylglycine.

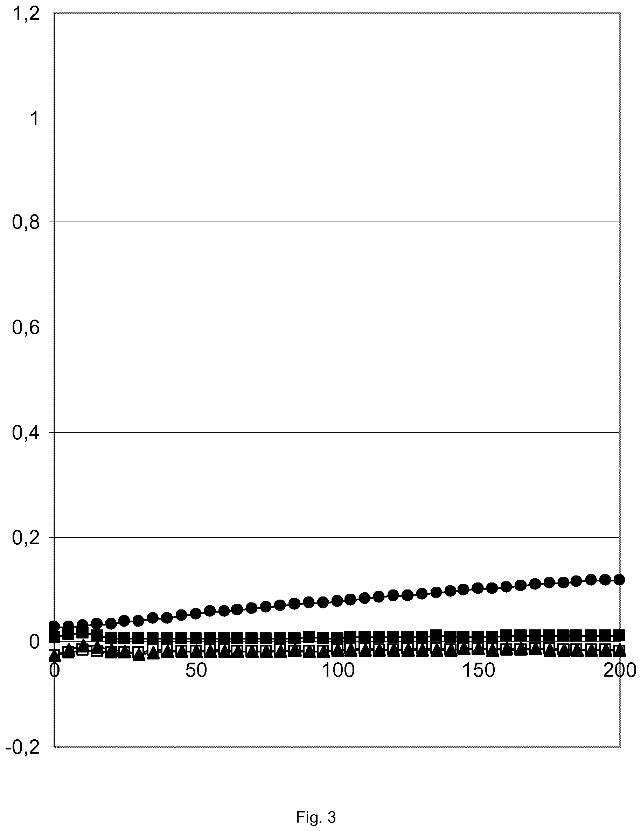

Figure 3 shows a graph for the absorption measurements of the PPi release assay with the single adenylation domain derived from VegA_M1_A. A very minor adenylation activity is determined for the amino acids L-hydroxyphenylglycine, while no activity was determined for D-hydroxyphenylglycine, D- and L-phenylalanine.

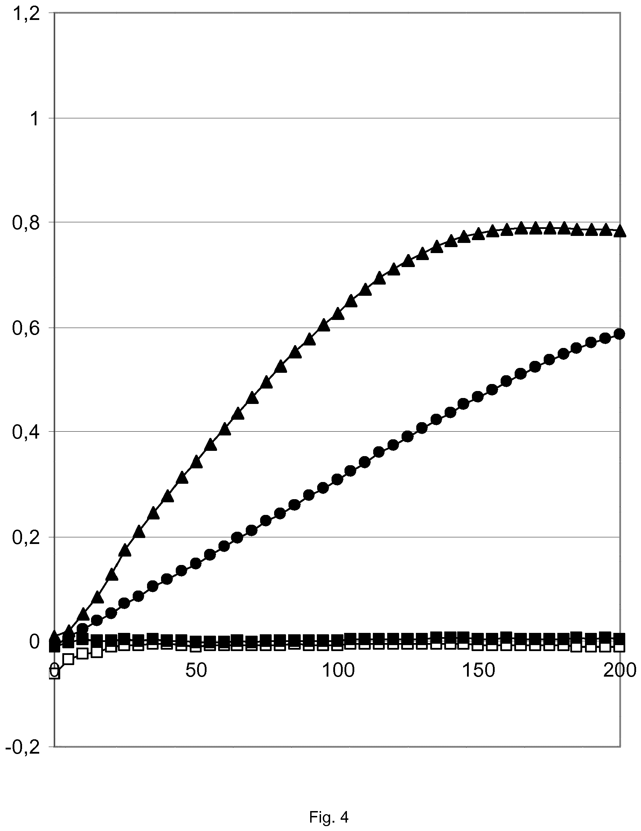

Figure 4 shows a graph for the absorption measurements of the PPi release assay with the single adenylation domain derived from VegA_M1_A co-purified with the MbtH-like protein Tcp13. A clear adenylation activity is determined for the amino acids L- and D-hydroxyphenylglycine, while no activity is determined for L- or D-phenylalanine. The graphs for the adenylation activity determinations of CepB_M2_A, BpsB_M2_A, Tcp11_M1_A, StaC_M2_A, or the adenylation thiolation didomain of Tcp9_M1_AT or the adenylation thiolation epimerisation tridomain of Veg8_M1_ATE all co-purified with the MbtH-like protein Tcp13 show the same results (data shown in Table 3). The graphs for the adenylation activity determinations of StaA_M1_A, and Dbv25_M1_A both co-purified with the MbtH-like protein VMbtH show the same results (data shown in Table 3).

Table 2 gives an overview on the adenylation activity determinations performed for single adenylation domains Tcp11_M1_A and VegA_M1_A, the adenylation thiolation didomain of Tcp9_M1_AT or the adenylation thiolation epimerisation tridomain of Veg8_M1_ATE all co-purified with the MbtH-like protein Tcp13, or Tcp17 or VMbtH given in amount of PPi formed per minute and mM of protein. In the adenylation activity determinations of ComB_M1_A, BpsB_M1_A, Teg7_M1_A all co-purified with the MbtH-like protein Tcp13, or Tcp17 or VMbtH no adenylation activity with D- or L-hydroxyphenylglycine, D- or L-phenylglycine D- (data shown in Table 3) or L-phenylalanine is determined. The adenylation activity determination of ComB_M1_A co-purified with the MbtH-like protein CMbtH derived from the same biosynthetic cluster as the A-domain confirmed its activity with L- or D-hydroxyphenylglycine; the adenylation activity determination of BpsB_M1_A co-purified with the MbtH-like protein BMbtH derived from the same biosynthetic cluster as the A-domain confirmed its activity with L-hydroxyphenylglycine, and the same specificity was determined in the adenylation activity determination of Teg7_M1_A co-purified with the MbtH-like protein TMbtH.

Table 3 gives a general overview on the adenylation activity determinations performed for the different amino acid substrates and the different combinations of either single adenylation domains, or the adenylation thiolation didomain of Tcp9_M1_AT or the adenylation thiolation epimerisation tridomain of Veg8_M1_ATE with the co-purified MbtH-like proteins Tcp13, or Tcp17 or VMbtH or CMbtH or BMbtH or TMbtH and the relative adenylation activities determined.

**Table 2: Adenylation activity determinations by PPi release assay of Tcp11_M1_A, Veg8M1_A, Tcp9_M1_AT and Veg8_M1_ATE in combination with MbtH like helper proteins Tcp13, Tcp17 or VMbtH.**

| Purified protein | Substrate | Formed PPi (mM/min/mM enzyme) | | |
|---|---|---|---|---|
| | | Tcp 13 | Tcp 17 | VMbtH |
| Tcp11_M1_A | D-HPG 1 mM | 0.66 | 0.63 | 0.86 |
| | D-HPG 0.1 mM | 0.08 | 0.11 | 0 |
| | L-HPG 1 mM | 1.03 | 1.04 | 1.54 |
| | L-HPG 0.1 mM | 0.80 | 0.95 | 1.38 |
| | D-PG 1 mM | 0 | 0.04 | 0 |
| | L-PG 1 mM | 0.17 | 0.23 | 0.09 |
| Veg8_M1_A | D-HPG 1 mM | 0.92 | 1.03 | 1.39 |
| | D-HPG 0.1 mM | 0.14 | 0.17 | 0.18 |
| | L-HPG 1 mM | 0.59 | 0.64 | 0.61 |
| | L-HPG 0.1 mM | 0.56 | 0.70 | 0.61 |
| | D-PG 1 mM | 0.01 | 0.02 | 0.02 |
| | L-PG 1 mM | 0.17 | 0.14 | 0.20 |
| Tcp9_M1_AT | D-HPG 1 mM | 5.28 | 4.63 | 8.44 |
| | D-HPG 0.1 mM | 2.07 | 1.71 | 3.72 |
| | L-HPG 1 mM | 1.16 | 1.34 | 1.40 |
| | L-HPG 0.1 mM | 1.18 | 1.20 | 1.23 |
| | D-PG 1 mM | 0.05 | 0.05 | 0.07 |
| | L-PG 1 mM | 1.32 | 1.44 | 2.32 |
| Veg8_M1_ATE | D-HPG 1 mM | 0.72 | 0.62 | 1.42 |
| | D-HPG 0.1 mM | 0.12 | 0.11 | 0.27 |
| | L-HPG 1 mM | 0.57 | 0.52 | 0.88 |
| | L-HPG 0.1 mM | 0.54 | 0.48 | 0.84 |
| | D-PG 1 mM | 0.01 | 0.01 | 0.02 |
| | L-PG 1 mM | 0.15 | 0.12 | 0.27 |

**Table 3**

| | | Substrates | | | | |
|---|---|---|---|---|---|---|
| Adenylation-domain | MbtH-like protein | L-HPG | D-HPG | L-PG | D-PG | L-Phe |
| StaA_M1_A | VMbtH | +++ | +++ | +++ | - | - |
| Dbv25_M1_A | VMbtH | +++ | +++ | +++ | - | - |
| StaC_M2_A | Tcp13 | ++ | ++ | - | - | - |
| Tcp11_M1_4 | Tcp13 / Tcp17 / VMbtH | +++ | +++ | +++ | - | - |
| Veg8_M1_A | Tcp13 / Tcp17 / VMbtH | +++ | +++ | +++ | - | - |
| BpsB_M2_A | Tcp13 | +++ | +++ | +++ | - | - |
| CepB_M2_A | Tcp13 | +++ | +++ | +++ | - | - |
| Tcp9_M1_AT | Tcp13 / Tcp17 / VMbtH | +++ | +++ | +++ | - | - |
| Veg8_M1_ATE | Tcp13 / Tcp17 / VMbtH | +++ | +++ | +++ | - | - |
| ComB_M1_A | Tcp13 / Tcp17 / VMbtH | - | - | - | - | - |
| BpsB_M1_A | Tcp13 / Tcp17 / VMbtH | - | - | - | - | - |
| Teg7_M1_A | Tcp13 / Tcp17 / VMbtH | - | - | - | - | - |
| ComB_M1_A | CMbtH | +++ | + | - | - | - |
| BpsB_M1_A | BMbtH | ++ | - | - | - | - |
| Teg7_M1_A | TMbtH | +++ | - | - | - | - |

## Claims

1. A method for the adenylation of 4-hydroxyphenylglycine or phenylglycine comprising contacting 4-hydroxyphenylglycine or phenylglycine with a non-ribosomal peptide synthetase and ATP, **characterized in that** an MbtH-like protein is present wherein said MbtH-like protein has SEQ ID NO: 18, SEQ ID NO: 19, SEQ ID NO: 20, SEQ ID NO: 30, SEQ ID NO: 31 or SEQ ID NO: 32 or a sequence that is at least 50% homologous to SEQ ID NO: 18, SEQ ID NO: 19 or SEQ ID NO: 20, SEQ ID NO: 30, SEQ ID NO: 31 or SEQ ID NO: 32 or has the amino acid code NXEXQXSXWP-X₅-PXGW-X₁₃-L-X₇-WTDXRP.

2. Method according to claim 1 wherein said non-ribosomal peptide synthetase comprises a first module M1 specific for 4-hydroxyphenylglycine and/or phenylglycine, a second module M2 specific for cysteine and a third module M3 specific for valine.

3. Method according to any one of claims 1 to 2 which is carried out in a eukaryotic microorganism.

4. Method according to claim 3 wherein said eukaryotic microorganism is Penicillium.

5. Method according to any one of claims 2 to 4 wherein said first module M1 comprises an adenylation domain chosen from the list consisting of SEQ ID NO: 1, SEQ ID NO: 2, SEQ ID NO: 3, SEQ ID NO: 4, SEQ ID NO: 5, SEQ ID NO: 6, SEQ ID NO: 7 and a sequence that is at least 50% homologous to SEQ ID NO: 1, SEQ ID NO: 2, SEQ ID NO: 3, SEQ ID NO: 4, SEQ ID NO: 5, SEQ ID NO: 6 or SEQ ID NO: 7.

6. A eukaryotic host cell comprising a non-ribosomal peptide synthetase, an isopenicillin N synthase and a polynucleotide allowing the expression of an MbtH-like protein wherein said MbtH-like protein has SEQ ID NO: 18, SEQ ID NO: 19, SEQ ID NO: 20, SEQ ID NO: 30, SEQ ID NO: 31 or SEQ ID NO: 32 or a sequence that is at least 50% homologous to SEQ ID NO: 18, SEQ ID NO: 19 or SEQ ID NO: 20, SEQ ID NO: 30, SEQ ID NO: 31 or SEQ ID NO: 32 or has the amino acid code NXEXQXSXWP-X₅-PXGW-X₁₃-L-X₇-WTDXRP. and wherein the MbtH-like protein has MbtH-like protein activity.

7. Eucaryotic host cell according to claim 6 which is *Penicillium chrysogenum, Acremonium chrysogenum* or *Aspergillus nidulans.*

## Patentansprüche

1. Verfahren zur Adenylierung von 4-Hydroxyphenylglycin oder Phenylglycin, bei dem man 4-Hydroxyphenylglycin bzw. Phenylglycin mit einer nicht ribosomalen Peptidsynthetase und ATP in Kontakt bringt, **dadurch gekennzeichnet, dass** ein MbtH-ähnliches Protein vorhanden ist, wobei das MbtH-ähnliche Protein SEQ ID NO: 18, SEQ ID NO: 19, SEQ ID NO: 20, SEQ ID NO: 30, SEQ ID NO: 31 oder SEQ ID NO: 32 oder eine Sequenz, die mit SEQ ID NO: 18, SEQ ID NO: 19, SEQ ID NO: 20, SEQ ID NO: 30, SEQ ID NO: 31 oder SEQ ID NO: 32 mindestens zu 50% homolog ist, oder den Aminosäurecode NXEXQXSXWP-X₅-PXGW-X₁₃-L-X₇-WTDXRP aufweist.

2. Verfahren nach Anspruch 1, wobei die nicht ribosomale Peptidsynthetase ein erstes für 4-Hydroxyphenylglycin und/oder Phenylglycin spezifisches Modul M1, ein zweites für Cystein spezifisches Modul M2 und ein drittes für Valin spezifisches Modul M3 umfasst.

3. Verfahren nach einem der Ansprüche 1 bis 2, welches in einem eukaryotischen Mikroorganismus durchgeführt wird.

4. Verfahren nach Anspruch 3, wobei es sich bei dem eukaryotischen Mikroorganismus um Penicillium handelt.

5. Verfahren nach einem der Ansprüche 2 bis 4, wobei das erste Modul M1 eine aus der aus SEQ ID NO: 1, SEQ ID NO: 2, SEQ ID NO: 3, SEQ ID NO: 4, SEQ ID NO: 5, SEQ ID NO: 6, SEQ ID NO: 7 und einer Sequenz, die mit SEQ ID NO: 1, SEQ ID NO: 2, SEQ ID NO: 3, SEQ ID NO: 4, SEQ ID NO: 5, SEQ ID NO: 6 oder SEQ ID NO: 7 zu mindestens 50% homolog ist, bestehenden Liste ausgewählte Adenylierungsdomäne umfasst.

6. Eukaryotische Wirtszelle, umfassend eine nicht ribosomale Peptidsynthetase, eine Isopenicillin-N-Synthase und ein Polynukleotid, welches die Expression eines MbtH-ähnlichen Proteins erlaubt, wobei das MbtH-ähnliche Protein SEQ ID NO: 18, SEQ ID NO: 19, SEQ ID NO: 20, SEQ ID NO: 30, SEQ ID NO: 31 oder SEQ ID NO: 32 oder eine Sequenz, die mit SEQ ID NO: 18, SEQ ID NO: 19 oder SEQ ID NO: 20, SEQ ID NO: 30, SEQ ID NO: 31 oder SEQ ID NO: 32 zu mindestens 50% homolog ist, oder den Aminosäurecode NXEXQXSXWP-X₅-PXGW-X₁₃-L-X₇-WTDXRP aufweist und wobei das MbtH-ähnliche Protein über die Aktivität des MbtH-ähnlichen Proteins verfügt.

7. Eukaryotische Wirtszelle nach Anspruch 6, bei der es sich um *Penicillium chrysogenum, Acremonium chrysogenum* oder *Aspergillus nidulans* handelt.

## Revendications

1. Procédé d'adénylation de 4-hydroxyphénylglycine ou de phénylglycine comprenant la mise en contact de 4-hydroxyphénylglycine ou de phénylglycine avec une peptide synthétase non ribosomique et de l'ATP, **caractérisé en ce qu'**une protéine de type MbtH est présente, dans lequel ladite protéine de type MbtH présente SEQ ID NO: 18, SEQ ID NO: 19, SEQ ID NO: 20, SEQ ID NO: 30, SEQ ID NO: 31 ou SEQ ID NO: 32 ou une séquence qui est au moins 50 % homologue à SEQ ID NO: 18, SEQ ID NO: 19 ou SEQ ID NO: 20, SEQ ID NO: 30, SEQ ID NO: 31 ou SEQ ID NO: 32 ou présente le code d'acides aminés NXEXQXSXWP-X₅-PXGW-X₁₃-L-X₇-WTDXRP.

2. Procédé selon la revendication 1, dans lequel ladite peptide synthétase non ribosomique comprend un premier module M1 spécifique pour la 4-hydroxyphénylglycine et/ou la phénylglycine, un deuxième module M2 spécifique pour la cystéine et un troisième module M3 spécifique pour la valine.

3. Procédé selon l'une quelconque des revendications 1 à 2, qui est conduit dans un micro-organisme eucaryote.

4. Procédé selon la revendication 3, dans lequel ledit micro-organisme eucaryote est Pénicillium.

5. Procédé selon l'une quelconque des revendications 2 à 4, dans lequel ledit premier module M1 comprend un domaine d'adénylation choisi dans la liste constituée de SEQ ID NO: 1, SEQ ID NO: 2, SEQ ID NO: 3, SEQ ID NO: 4, SEQ ID NO: 5, SEQ ID NO: 6, SEQ ID NO: 7 et une séquence qui est au moins 50 % homologue à SEQ ID NO: 1, SEQ ID NO: 2, SEQ ID NO: 3, SEQ ID NO: 4, SEQ ID NO: 5, SEQ ID NO: 6 ou SEQ ID NO: 7.

6. Cellule hôte eucaryote comprenant une peptide synthétase non ribosomique, une isopénicilline N synthase et un polynucléotide permettant l'expression d'une protéine de type MbtH, dans laquelle ladite protéine de type MbtH présente SEQ ID NO: 18, SEQ ID NO : 19, SEQ ID NO: 20, SEQ ID NO: 30, SEQ ID NO: 31 ou SEQ ID NO: 32 ou une séquence qui est au moins 50 % homologue à SEQ ID NO: 18, SEQ ID NO: 19 ou SEQ ID NO: 20, SEQ ID NO: 30, SEQ ID NO: 31 ou SEQ ID NO: 32 ou présente le code d'acides aminés NXEXQXSXWP-X₅-PXGW-X₁₃-L-X₇-WTDXRP, et dans laquelle la protéine de type MbtH possède une activité de protéine de type MbtH.

7. Cellule hôte eucaryote selon la revendication 6 qui est *Pénicillium chrysogenum, Acremonium chrysogenum* ou *Aspergillus nidulans.*
